(19) Europäisches Patentamt
European Patent Office
Office européen des brevets

(11) **EP 3 128 330 B1**

(12) **EUROPEAN PATENT SPECIFICATION**

(45) Date of publication and mention
of the grant of the patent:
**06.05.2020 Bulletin 2020/19**

(51) Int Cl.:
***G01N 21/51*** *(2006.01)*     ***G01N 21/27*** *(2006.01)*
***G01N 21/59*** *(2006.01)*     ***G16H 10/40*** *(2018.01)*
*G01N 33/49* *(2006.01)*     *G01N 21/82* *(2006.01)*
*G01N 33/86* *(2006.01)*     *G01N 21/13* *(2006.01)*
*G16H 50/20* *(2018.01)*

(21) Application number: **15773133.2**

(22) Date of filing: **09.03.2015**

(86) International application number:
**PCT/JP2015/056841**

(87) International publication number:
**WO 2015/151732 (08.10.2015 Gazette 2015/40)**

(54) **AUTOMATIC ANALYTICAL APPARATUS**

AUTOMATISCHE ANALYTISCHE VORRICHTUNG

APPAREIL ANALYTIQUE AUTOMATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.04.2014 JP 2014075478**

(43) Date of publication of application:
**08.02.2017 Bulletin 2017/06**

(73) Proprietor: **Hitachi High-Tech Corporation
Minato-ku
Tokyo 105-6409 (JP)**

(72) Inventors:
• **TARUMI, Shinji
Tokyo 100-8280 (JP)**
• **ADACHI, Sakuichiro
Tokyo 100-8280 (JP)**
• **YABUTANI, Chie
Tokyo 105-8717 (JP)**
• **MAKINO, Akihisa
Tokyo 105-8717 (JP)**

(74) Representative: **MERH-IP Matias Erny Reichl
Hoffmann
Patentanwälte PartG mbB
Paul-Heyse-Strasse 29
80336 München (DE)**

(56) References cited:
EP-A1- 2 434 292     EP-A1- 2 594 944
EP-A1- 2 711 713     EP-A1- 2 927 694
EP-A1- 2 982 988     WO-A1-2012/008324
JP-A- 2004 012 442     JP-A- 2004 508 832
JP-A- 2005 201 843     JP-A- 2006 337 125
JP-A- 2006 349 684     JP-A- 2010 271 095

## Description

Technical field

The present invention relates to an automatic analytical apparatus.

Background art

A blood coagulation test is performed for several purposes, such as pathology recognition of a congealing fibrinolysis system, diagnosis of disseminated intravascular coagulation (DIC), confirmation of a thrombotic therapeutic effect, and diagnosis of hemophilia. Conventionally, the blood coagulation test has been performed by visually recognizing fibrin deposition that is an end point of the blood coagulation reaction. However, since the 1960s, a blood coagulation analytical apparatus developed for the purpose of test throughput improvement and high accuracy has been widely used in daily testing.

For detection of fibrin deposition in a blood coagulation analytical apparatus, a measurement scheme such as an electric resistance detection scheme, an optical detection scheme, or a mechanical scheme is mainly used. In particular, since the optical detection scheme is a non-contact measurement scheme in which foreign matter does not come into direct contact with a sample during a coagulation reaction, time scheme has been widely used. The optical detection scheme includes two types including a transmitted light detection scheme of measuring a change in transmitted light when an agglomerate is generated in a reaction container, and a scattered light detection scheme of detecting scattered light. Any of the schemes is a scheme of detecting the progress of a coagulation reaction as a reaction curve on the basis of a time-series light intensity change and realizing high-precision analysis.

The optical detection scheme is characterized by non-contact and high precision as described above, but an unstable reaction curve may be measured in the blood coagulation reaction, unlike other biochemical reactions. Causes thereof may include a coagulation reaction that is a complex multi-step reaction in which a plurality of causes interact with each other, mixing of foreign matter such as air bubbles at the time of stirring in a reaction container during measurement, and the like. The unstable reaction curve caused by the former cause includes a two-step reaction in which a reaction rate once decreases and then increases again. Further, the unstable reaction curve caused by the latter measurement abnormality may include a drift reaction in which a rate does not equal zero in a second half of the reaction, a jump abnormality in which the light intensity greatly changes in a short time in a reaction curve, a noise in which the light intensity temporarily fluctuates up and down, or the like.

Conventionally, in a case in which the abnormality occurs, it is necessary for a worker in the field to visually confirm a reaction curve of each sample, determine and classify an abnormality, and examine a subsequent countermeasure such as re-testing or checking of the apparatus. However, in daily test work, it is difficult to visually confirm all measured reaction curves, and in particular, a less accurate test is likely to be performed as a result of overlooking an abnormality for data for which a normal test value has been once measured. Therefore, an automatic analytical apparatus and a processing method of automatically detecting the abnormality as described above included in the reaction curve and notifying a worker of the abnormality has been proposed. For example, in PTL 1, a blood coagulation reaction analysis method of setting a check area for a specific change amount and time and setting a threshold value for a light intensity, a time, and a reaction rate to detect an abnormality has been proposed. PTL 2 shows an automatic analysis device according to the preamble of claim 1, which mixes a sample and a reagent to measure temporal change of a mixed solution.

In PTL 3 an automated analyzer and an automatic analysis method determine abnormality based on reaction process data obtained. The reaction process data is approximated by a function, and shape feature quantities indicating features of a shape of a curve section are calculated. The obtained shape feature quantities are used to determine abnormality.

In PTL 4 an automatic analyzer obtains multiple measurement data points during the progress of reaction of a mixed solution. With these measurement results the analyzer selects at least one approximation formula; generates an approximation curve from the measurement data points; calculates a shape descriptor from the approximation curve; and judges abnormalities based on the shape descriptor.Citation List

Patent Literature

PTL 1: JP-A-2003-169700
PTL 2: EP 2 711 713 A1
PTL 3: EP 2 434 292 A1
PTL 4: EP 2 594 944 A1

Summary of Invention

Technical Problem

However, in the method shown in PTL 1, information on the detected abnormality could not be appropriately classified. Further, in a coagulation reaction process curve acquired using an optical detection scheme, since a large difference is generated in a change in a rate of reaction or a light intensity before and after the reaction due to content of a substance contained in a sample, the abnormality cannot be accurately detected from a gradual reaction curve of progress of a coagulation reaction. Thus, in the conventionally disclosed technology, an abnormal reaction process curve acquired in a blood coagulation reaction having a feature to draw a different curve for each sample cannot be accurately detected and classified.

An object of the present invention is to provide a technology capable of accurately detecting and classifying an abnormal reaction process curve in an automatic analytical apparatus that analyzes components contained in a sample.

Solution to Problem

The present invention is defined in claim 1. In the description of the invention, which follows, embodiments of the present invention are only those arrangements, which comprise all features of claim 1. Any other arrangement not falling under the terms of claim 1 is merely an example, which is useful for understanding the present invention, even if it may be referred to as "embodiment".

In order to solve the above problem, for example, a configuration described in claims is adopted. This application includes a plurality of means for solving the above problem. For example, provided is an automatic analytical apparatus, including: a reaction container for mixing a sample with a reagent to react the sample to the reagent; a measurement unit that irradiates a reaction solution in the reaction container with light and measures the intensity of transmitted light or scattered light; a control unit that processes time-series light intensity data obtained through the measurement in the measurement unit; a storage unit that stores one or more approximation functions each approximating to a time-series change in the light intensity data; and an output unit that outputs a processing result of the control unit, wherein the control unit selects any one of the approximation functions stored in the storage unit, calculates an approximate curve indicating a time-series change in the light intensity data using the selected approximation function, calculates deviation feature information based on deviation information between the light intensity data and the approximate curve, and detects and classifies abnormality included in the light intensity data using the deviation feature information, and the output unit outputs information on the detected and classified abnormality.

Advantageous Effects of Invention

According to the present invention, it is possible to accurately detect and classify an abnormal reaction process curve in an automatic analytical apparatus that analyzes components contained in a sample. Additional characteristics relevant to the present invention are apparent from description of this specification and the accompanying drawings. Further, problems, configurations, and effects other than those described above will become apparent from description of the following embodiments.

Brief Description of Drawings

[Fig. 1] Fig. 1 is a configuration diagram of an automatic analytical apparatus according to a first embodiment.
[Fig. 2] Fig. 2 is a diagram illustrating a process flow of detecting and classifying abnormal light intensity data in the first embodiment.
[Fig. 3] Fig. 3 is a diagram illustrating a process flow of an abnormality classification process in step S208 of Fig. 2.
[Fig. 4] Fig. 4 is a diagram illustrating a process flow of detecting and classifying abnormal light intensity data in a second embodiment.
[Fig. 5] Fig. 5 is a diagram illustrating a process flow of detecting and classifying abnormal light intensity data in a third embodiment.
[Fig. 6] Fig. 6 is a diagram in a case in which a process of step S203 in Fig. 2 is applied to light intensity data including a jump abnormality.
[Fig. 7] Fig. 7 is a diagram in which an error between time-series light intensity data and an approximate curve illustrated in Fig. 6 is calculated and plotted for each data point of the time-series light intensity data.

[Fig. 8] Fig. 8 is a diagram in a case in which a process of step S203 in Fig. 2 is applied to light intensity data including a drift abnormality.

[Fig. 9] Fig. 9 is a diagram in which an error between time-series light intensity data and an approximate curve illustrated in Fig. 8 is calculated and plotted for each data point of the time-series light intensity data.

[Fig. 10] Fig. 10 is a diagram in a case in which a process of step S203 in Fig. 2 is applied to light intensity data including a two-step reaction abnormality.

[Fig. 11] Fig. 11 is a diagram in which an error between time-series light intensity data and an approximate curve illustrated in Fig. 10 is calculated and plotted for each data point of the time-series light intensity data.

[Fig. 12] Fig. 12 is a diagram in a case in which a process of step S203 is applied to light intensity data having an abnormality including noise.

[Fig. 13] Fig. 13 is a diagram in which an error between time-series light intensity data and an approximate curve illustrated in Fig. 12 is calculated and plotted for each data point of the time-series light intensity data.

[Fig. 14] Fig. 14 is a diagram illustrating an output screen example of an operation computer.

[Fig. 15] Fig. 15 is a diagram in a case in which processes of step S203 and step S205 in Fig. 2 are applied to time-series light intensity data having a drift abnormality respectively acquired from two samples.

[Fig. 16] Fig. 16 is a diagram in which an error between time-series light intensity data 1501 and an approximate curve 1502 illustrated in Fig. 15 and an error between time-series light intensity data 1503 and an approximate curve 1504 are calculated and plotted for each data point of the time-series light intensity data 1501 and each data point of the time-series light intensity data 1503.

[Fig. 17] Fig. 17 is a diagram in a case in which step S401 in Fig. 4 is applied to time-series error data 1601 and 1602 illustrated in Fig. 16.

[Fig. 18] Fig. 18 is a diagram in a case in which step S401 in Fig. 4 is applied to time-series error data 1701 and 1702 illustrated in Fig. 17.

[Fig. 19] Fig. 19 is a diagram in a case in which a process of step S203 is applied to time-series light intensity data during a reaction having a jump abnormality.

[Fig. 20] Fig. 20 is a diagram in which an error between time-series light intensity data and an approximate curve illustrated in Fig. 19 is calculated and plotted for each data point of the time-series light intensity data.

Description of Embodiments

Hereinafter, embodiments of the present invention will be described with reference to the accompanying drawings. The accompanying drawings show specific embodiments according to a principle of the present invention, but these are for the purpose of understanding of the present invention and are never used to restrictively construe the present invention.

[First Embodiment]

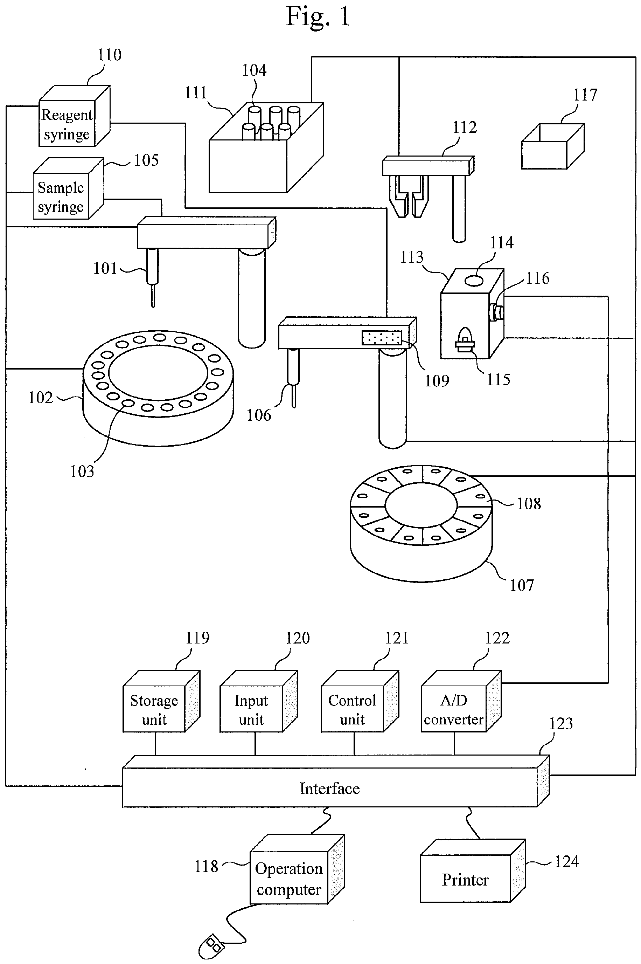

Fig. 1 is a diagram schematically illustrating a configuration of an automatic analytical apparatus according to the present invention. The automatic analytical apparatus analyzes a component contained in a sample from blood, and particularly analyzes a blood coagulation reaction. Since functions of respective unit are known, detailed description thereof will be omitted.

The automatic analytical apparatus according to the present invention is configured to aspirate a sample in a sample container 103 disposed in a sample disk 102 rotating left or right using a sample dispensing unit 101 and discharge the sample into a reaction container 104. The sample dispensing unit 101 performs a sample aspirating operation and a sample discharge operation according to an operation of a sample syringe pump 105. A reagent dispensing unit 106 is configured to aspirate a sample in a reagent container 108 disposed in a reagent disk 107 and discharge the same into the reaction container 104 according to an operation of a reagent syringe pump 110. A reagent heating unit 109 is built into the reagent dispensing unit 106.

The reaction container 104 is stored in a reaction container stock unit 111. A reaction container transport unit 112 performs transport and installation of the reaction containers 104. The reaction container 104 is held by the reaction container transport unit 112 from the reaction container stock unit 111, and the reaction container transport unit 112 installs the held reaction container 104 into a reaction container installation unit 114 of a detection unit 113 through rotational movement. In the reaction container installation unit 114, a dent is provided so that the reaction container 104 can be placed, and the reaction container 104 can be inserted into the dent. Here, the number of reaction container installation units 114 is at least one, and the apparatus includes at least one detection unit 113.

Next, a flow of measurement will be described. First, an analysis items to be analyzed for each sample is input from an input unit 120 such as a keyboard, or an operation computer 118. An operation of the detection unit 113 is controlled by a control unit 121. The sample in the sample container 103 disposed in the sample disk 102 is aspirated by the sample dispensing unit 101, and dispensed into the reaction container 104 placed into the reaction container installation unit 114 of the detection unit 113. Then, the reagent is aspirated from the reagent containers 108 disposed in the reagent disk 107 by the reagent dispensing unit 106 as well, heated to an appropriate temperature by the reagent heating unit 109, and dispensed into the reaction container 104. A blood coagulation reaction is started immediately by pressure of this reagent discharge.

The reaction container 104 is irradiated with light from a light source 115, and scattered light that is scattered in a reaction solution in the reaction container 104 or transmitted light that is transmitted in the reaction solution is detected by the detector 116, such as a photodiode. A photometric signal measured by the detector 116 passes through an A/D converter 122 and is taken as time-series light intensity data into the control unit 121 via an interface 123. In the control unit 121, a coagulation time is calculated from the time-series light intensity data. An output result of the control unit 121 is printed out by a printer 124 via the interface 123 or output on the screen of the operation computer 118, and is stored in a storage unit 119 realized by a RAM, a hard disk, or the like. The reaction container 104 for which the photometry has ended is held by the reaction container transport unit 112, and discarded into a reaction container discardment unit 117.

Hereinafter, a process of the control unit 121 will be described. The control unit 121 detects and classifies unique abnormality in the blood coagulation reaction. Hereinafter, an abnormal reaction curve generated in the blood coagulation reaction will first be described, and then, a process procedure will be described.

A blood coagulation reaction occurring in PT, APTT, Fbg, or the like performed as a blood coagulation test is a biological reaction in which an agglomerate is generated through a complicated reaction system. In a case in which this aggregate is tested using an optical detection scheme, a reaction curve therefor is generally a growth type curve. However, in a case in which a sample to be used for the test has an abnormality or in a case in which a foreign matter such as air bubbles at the time of stirring is mixed in the reaction container during measurement, a reaction curve having a different shape is likely to be obtained. For example, according to the sample, a two-step reaction curve in which a change amount of the light intensity gradually increases, decreases, then and, increases again may be obtained. In addition, air bubbles or other related particles may be often caught in a mixture solution and pass through a measurement area, and the intensity of light may temporarily change. As a result, a jump abnormality in which the intensity of light greatly changes in a short period of time in the reaction curve, noise in which the light intensity temporarily fluctuates up and down, or the like may be generated. Further, in a second half of the reaction curve, a drift abnormality in which a reaction rate is not converged to zero and the light intensity continuously changes may be generated.

All of the abnormalities are characterized by a reaction curve having a shape deviating from an ideal growth-type reaction curve. Therefore, on the basis of the abnormality characteristic, the control unit 121 of the present invention calculates a growth-type approximate curve from the time-series light intensity data, calculates the deviation feature information indicating a degree of deviation between the time-series light intensity data and the approximate curve, and detects and classifies the abnormality using the deviation feature information. Accordingly, abnormalities widely known to occur in a coagulation test, including the above-described abnormalities, can be detected and classified.

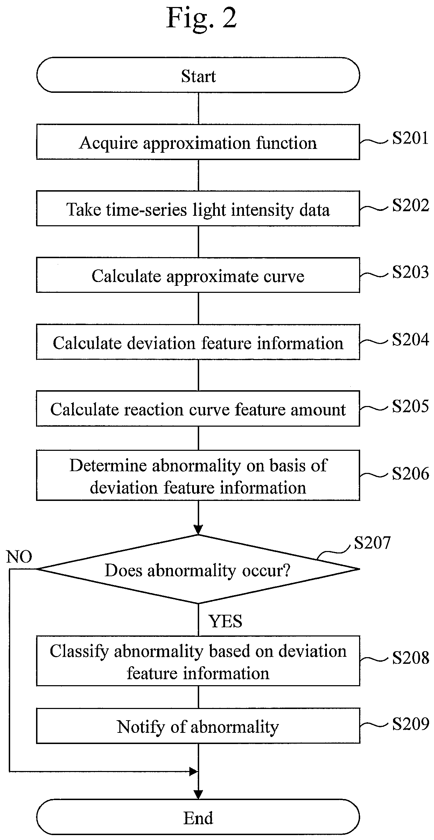

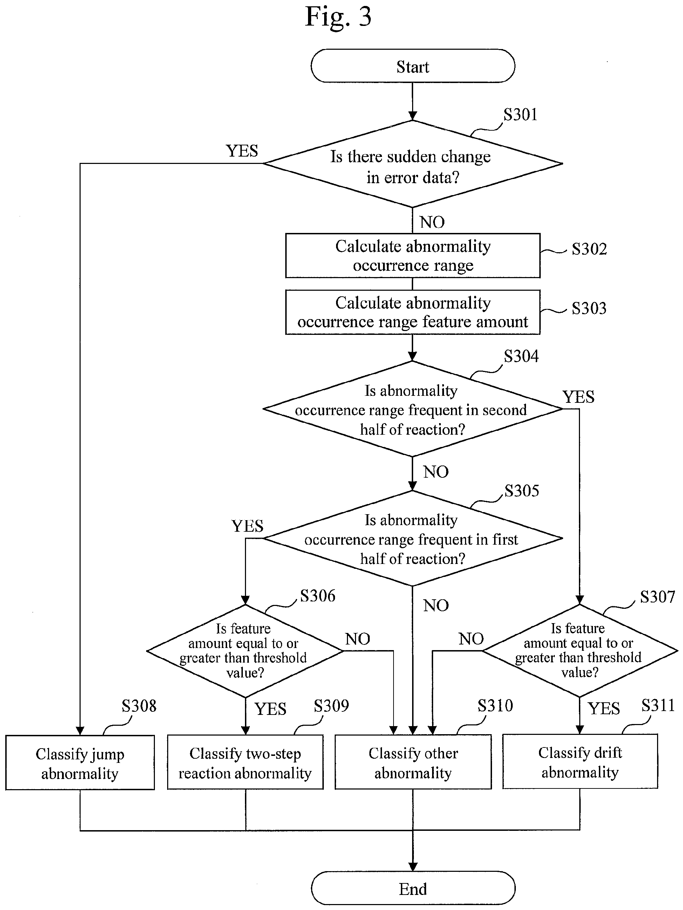

Hereinafter, a process flow in the control unit 121 will be described with reference to Figs. 2 and 3. Fig. 2 is a diagram illustrating process steps of a portion related to abnormality detection and classification in the control unit 121. Fig. 3 is a diagram illustrating steps of a process performed in S208 in Fig. 2. Hereinafter, the respective steps in Fig. 2 will be described.

First, in step S201, the control unit 121 selects and acquires an optimal approximation function corresponding to a combination of test items and reagents from among a plurality of approximation functions for approximating a time change in light intensity stored in the storage unit 119 in advance. For example, the combination of test items and reagents and the approximation function corresponding thereto may be defined in advance, and the control unit 121 may automatically select an optimal approximation function according to the definition. For example, a logistic function shown in Equation 1 below is considered as the approximation function.

$$y = y_{max} - y_{range} / (1 + exp\ (\alpha1(t - \alpha2)))$$

In Equation 1, t indicates time, y indicates a light intensity value, and $y_{max}$, $y_{range}$, $\alpha1$, and $\alpha2$ are parameters. For example, $y_{max}$ is a maximum value of y, $y_{range}$ is a range of a value of y (maximum value ($y_{max}$) of y - minimum value ($y_{min}$) of y).

In step S202, the control unit 121 takes, as the time-series light intensity data, light/current conversion data detected by the detector 116 in a predetermined period, via the A/D converter 122 and the interface 123. The control unit 121 monitors the taken time-series light intensity data, and proceeds to step S203 if the coagulation reaction has ended. For a coagulation reaction end determination, a generally widely known method may be used. For example, it can be determined that the coagulation reaction has completed using a scheme of setting a threshold value for a

5

measurement time of the time-series light intensity data or a scheme of setting a threshold value for a recent light intensity value or the amount of change in the light intensity value.

In step S203, the control unit 121 calculates s value of a parameter in the approximation function so that a difference between an approximate curve of a time-light intensity value expressed by the approximation function selected in step S201, and the time-series light intensity data is as small as possible. For example, the control unit 121 determines the value of the parameter in the approximation function so that a square error between the time-series light intensity data and the light intensity calculated by the approximation function is as small as possible. As a scheme of calculating the parameter value, for example, a combination of a least square calculation method and a steepest descent method, a Newton's method, or the like may be used. In this step, an approximate curve which best approximates the light intensity data is obtained. That is, the approximation function stored in the storage unit 119 functions as an initial value for obtaining the approximate curve indicating the light intensity data.

In step S204, the control unit 121 calculates deviation feature information between the approximate curve calculated in step S203 and the time-series light intensity data. Here, the deviation feature information is information representing a degree of approximation of the approximate curve to the time-series light intensity data, and is information that is calculated as a scalar value or a vector value. As the deviation feature information, for example, time-series error data obtained by calculating an error between the time-series light intensity data and the approximate curve for each time-series light intensity data point may be used. A value calculated as a value having a sign, such as a value calculated by subtracting a value of the light intensity calculated using the approximate curve from a value of the time-series light intensity data, rather than an absolute value, may be used as the error. In this embodiment below, a case in which the time-series error data calculated by subtracting the value of the light intensity calculated using the approximate curve from the value of the time-series light intensity data is used as the deviation feature information will be described by way of example, but the deviation feature information to be calculated is not limited thereto.

In step S205, the control unit 121 calculates the feature amount from the reaction curve. Here, the reaction curve includes time-series light intensity data taken in step S202 and the approximate curve calculated in step S203. As the feature amount, for example, the tentative coagulation time calculated on the basis of the approximate curve may be used. Hereinafter, the example in which the coagulation time calculated on the basis of the approximate curve is used will be described. However, a coagulation time obtained from the time-series light intensity data (that is, raw measurement data) taken in step S202 may also be used as the feature amount. As a scheme of calculating the coagulation time, any widely known method may be used. For example, a method of calculating differential data by calculating an inter-data difference of approximate curves and calculating a peak position of the differential data as a coagulation time may be considered.

In step S206, the control unit 121 determines whether an abnormality is included in the time-series light intensity data on the basis of the deviation feature information calculated in step S204. As an example of a determination method, a method of setting a threshold value of an error for time-series error data calculated as deviation feature information, calculating the number of data points having an error equal to or greater than the threshold value, and comparing the calculated number of data points with a threshold value of the number of abnormality data points to determine an abnormality may be used.

For the threshold value of the error, a predetermined value may be used. Further, the threshold value of the error may be determined on the basis of the number of data points of the time-series light intensity data taken in step S202 and a range of light intensity value. Further, the threshold value of the error may be determined on the basis of the feature amount of the approximate curve calculated in step S205. Further, the threshold value of the error may be determined on the basis of the deviation feature information calculated in step S204 and, for example, on the basis of a value such as a variance or an average of data of a subset of the time-series error data.

For a threshold value of the number of abnormality data points, a predetermined value may be used. Further, the threshold value of the number of abnormality data points may be determined on the basis of the number of data points of the time-series light intensity data taken in step S202. Further, the threshold value of the number of abnormality data points may be determined on the basis of the feature amount of the approximate curve calculated in step S205. Further, the threshold value of the number of abnormality data points may be calculated on the basis of the deviation feature information calculated in step S204.

In step S207, the control unit 121 determines a subsequent process step on the basis of the result of the abnormality determination in step S206. In a case in which it is not determined that the abnormality is included in the time-series light intensity data in step S206, the process ends. In a case in which it is determined that the abnormality is included in the time-series light intensity data, the process proceeds to step S208.

In step S208, the control unit 121 classifies the type of abnormality that is included in the time-series light intensity data on the basis of the deviation feature information calculated in step S204, and reads an abnormality code corresponding to the classified abnormality from the storage unit 119. Here, the abnormality code is key information assigned to each type of abnormality that has been classified in advance. In the storage unit 119, the abnormality code, and information on the abnormality corresponding to the abnormality code are stored in association with each other. As an example of the classification method, a determination process is repeatedly applied to the deviation feature information calculated in step S204 on the basis of a decision tree stored in the storage unit 119 in advance, and the type of abnormality included in the time-series light intensity data is classified. An example of process steps of the abnormality classification process using the decision tree for the time-series error data will be described below with reference to Fig. 3.

In step S209, the control unit 121 reads information on the abnormality stored in the storage unit 119 using the abnormality code calculated in step S208 as a key, and outputs the information to the operation computer 118 or the printer 124. Here, the information on the abnormality includes a name of the abnormality, the type of abnormality, a method of coping with the abnormality (a countermeasure corresponding to the type of abnormality), and the like. Therefore, a rapid transition to next countermeasure such as re-test can be performed and efficiency of test work can be realized. Further, the information to be output may include basic information such as a sample number or a test item, information on the time-series light intensity data taken in step S202, information on the approximate curve calculated in step S203, the deviation feature information calculated in step S204, information on the feature amount of the reaction curve calculated in step S205, information on whether the abnormality determined in step S206 is included, and the like. Further, the information to be output may be stored in the storage unit 119.

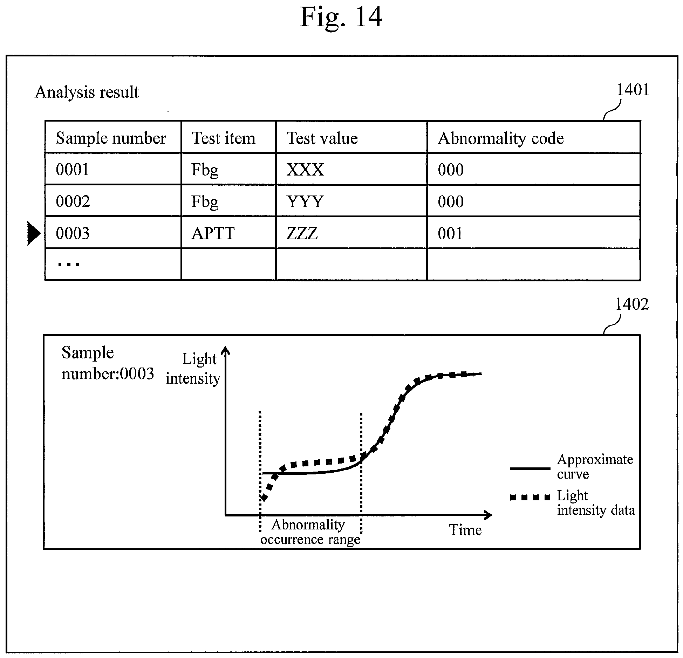

Fig. 14 illustrates an example of an output screen of the operation computer 118. The screen of Fig. 14 includes a basic information display portion 1401, and a graph display portion 1402. Information such as a sample number, a test item, and an abnormality code is displayed in the basic information display portion 1401. A graph representing a reaction curve and an approximate curve of time-series light intensity data corresponding to a row selected in the basic information display portion 1401 is displayed in the graph display portion 1402. In the example of Fig. 14, a row of the sample number "0003" of the basic information display portion 1401 is selected, and the graph representing the reaction curve and the approximate curve of the time-series light intensity data corresponding thereto and a range of abnormality occurrence (abnormality occurrence range) are displayed in the graph display portion 1402.

Next, an example of process steps of the abnormality classification process in step S208 will be described with reference to Fig. 3. The abnormality classification process will be described as an example of a process that is effective when the control unit 121 calculates time-series error data as deviation feature information in step S204.

First, in step S301, the control unit 121 determines whether there is a sudden change in the time-series error data. The sudden change in the error can be detected by setting a threshold value in the amount of change between an error of a certain data point and an error of a data point acquired subsequently. As the threshold value, a preset threshold value may be used. In this case, whether a sign of a data point at which the amount of change is calculated is different may be added to a determination criterion. In a case in which it is determined that there is a sudden change, the process proceeds to step S308. In a case in which it is not determined that there is a sudden change, the process proceeds to step S302.

In step S302, the control unit 121 calculates a range in the time-series in which abnormality occurs in the time-series light intensity data using the time-series error data. The range is hereinafter referred to as an abnormality occurrence range. The abnormality occurrence range is indicated by a vector having the same length as the time-series error data, which is a vector representing whether each data point is abnormal using 2 values including 1 and 0. Further, the abnormality occurrence range may also be indicated by defining a start position and an end point of a set of data points determined to be abnormal. As a method of calculating the abnormality occurrence range, for example, a method of comparing the value of each data point in the time-series error data with a preset threshold value, determining a data point having a value equal to or greater than a threshold value as an abnormality data point, and then, calculating a range may be used.

In step S303, the control unit 121 calculates a feature amount regarding a shape of the time-series error data in the abnormality occurrence range calculated in step S302. Hereinafter, an example of the feature amount will be described. All abnormality occurrence ranges calculated in step S302 may be used as the abnormality occurrence range in the following description or the abnormality occurrence range is divided into ranges having continuous data points, and some of the ranges may be used. Further, the abnormality occurrence range may be divided on the basis of a sign of the error data included in the abnormality occurrence range, and some of divisions may be used.

A first example of the feature amount will be described. The number of data points included in the abnormality occurrence range is the feature amount.

A second example of the feature amount will be described. A value that can be calculated from the error, such as an average value, a dispersion value, a total value, a maximum value, a median value, a quartile value, an error value of a start position, or an error value of an end position of the time-series error data within the abnormality occurrence range may be used as the feature amount. In this case, a time at which the maximum value, the median value, or the quartile value is taken may be used as the feature amount. Further, a value calculated by combining the plurality of values and using four arithmetic operations may be used as the feature amount.

A third example of the feature amount will be described. A parameter obtained by approximating the approximation function stored in the storage unit 119 to the error data in the error occurrence range, and an index indicating a degree of the approximation are used as feature amounts. As the approximation function, for example, a polynomial equation or an equation based on a trigonometric function may be used. As the degree of the approximation, a least square error sum, a determination coefficient, or the like may be used.

A fourth example of the feature amount will be described. In each range obtained by dividing the abnormality occurrence range, each feature amount described above may be calculated, and a value calculated by combining the calculated feature amounts and using four arithmetic operations may be set as a feature amount.

In step S304, the control unit 121 determines whether the abnormality occurrence range calculated in step S302 is frequent in the second half of the reaction. The determination can be performed using the feature amount of the approximate curve calculated in step S205. For example, the control unit 121 may determine whether the abnormality occurrence range is frequent after the tentative coagulation time on the basis of the tentative coagulation time calculated in step S205. The abnormality occurrence range may appear in both of a time range before the tentative coagulation time and a time range after the tentative coagulation time. Therefore, through the above determination, it is determined whether the abnormality occurrence range is frequent in the second half of the reaction. In a case in which the abnormality occurrence range is frequent in the second half, the process proceeds to step S307. In a case in which the abnormality occurrence range is not frequent in the second half, the process proceeds to step S305.

The determination in step S304 is not limited thereto. For example, it may be determined whether the abnormality occurrence range is only in the second half of the reaction. The abnormality occurrence range may appear only in any one of the time range before the tentative coagulation time and the time range after the tentative coagulation time. In this case, it is possible to determine whether the abnormality occurrence range is only in the second half of the reaction by comparing the tentative coagulation time calculated in step S205 with a start position of the abnormality occurrence range .

In step S305, the control unit 121 determines whether the abnormality occurrence range calculated in step S302 is frequent in the first half of the reaction. The determination can be performed using the feature amount of the approximate curve calculated in step S205. For example, the control unit 121 may determine whether the abnormality occurrence range is frequent before the tentative coagulation time on the basis of the tentative coagulation time calculated in step S205. The abnormality occurrence range may appear in both of a time range before the tentative coagulation time and a time range after the tentative coagulation time. Therefore, through the above determination, it is determined whether the abnormality occurrence range is frequent in the first half of the reaction. In a case in which the abnormality occurrence range is frequent in the first half, the process proceeds to step S306. In a case in which the abnormality occurrence range is not frequent in the first half, the process proceeds to step S310.

The determination in step S305 is not limited thereto. For example, it may be determined whether the abnormality occurrence range is only in the first half of the reaction. The abnormality occurrence range may appear only in any one of the time range before the tentative coagulation time and the time range after the tentative coagulation time. In this case, it is possible to determine whether the abnormality occurrence range is only in the first second half of the reaction by comparing the tentative coagulation time calculated in step S205 with an end position of the abnormality occurrence range.

In step S306, the control unit 121 compares the feature amount calculated in step S303 with a predetermined threshold value, and determines whether the type of abnormality is a two-step reaction abnormality. In a case in which the feature amount is equal to or greater than the threshold value, the process proceeds to step S309, and in a case in which the feature amount is not equal to or greater than the threshold value, the process proceeds to step S310. As the feature amount used for the determination, for example, a total value of errors in the abnormality occurrence range or a percentage in the abnormality occurrence range of a total value of errors in the respective abnormality occurrence ranges may be used.

In step S307, the control unit 121 compares the feature amount calculated in step S303 with a predetermined threshold value and determines whether the type of abnormality is a drift abnormality. In a case in which the feature amount is equal to or greater than the threshold value, the process proceeds to step S311, and in a case in which the feature amount is not equal to or greater than the threshold value, the process proceeds to step S310. As the feature amount used for the determination, for example, a parameter obtained by approximating a primary equation to the abnormality occurrence range may be used.

In step S308, the control unit 121 classifies an abnormality detected in step S206 as jump abnormality, and reads an abnormality code corresponding to the jump abnormally and information on the jump abnormality from the storage unit 119.

In step S309, the control unit 121 classifies the abnormality detected in step S206 as a two-step reaction abnormality, and reads an abnormality code corresponding to the two-step reaction abnormality and information on the two-step reaction abnormality from the storage unit 119.

In step S310, the control unit 121 classifies the abnormality detected in step S206 as other abnormality, and reads an abnormality code corresponding to the other abnormality and information on the other abnormality from the storage unit 119. Thus, by setting the classification of the other abnormality, it is possible to recognize an abnormality that has not been recognized heretofore. In this case, a threshold determination different from S306 and S307 may be further performed on the feature amount, and another abnormality may be further classified. An example of the other abnormality may include a reaction curve including noise.

In step S311, the control unit 121 classifies the abnormality detected in step S206 as a drift abnormality, and reads an abnormality code corresponding to the drift abnormality and information on the drift abnormality from the storage unit 119.

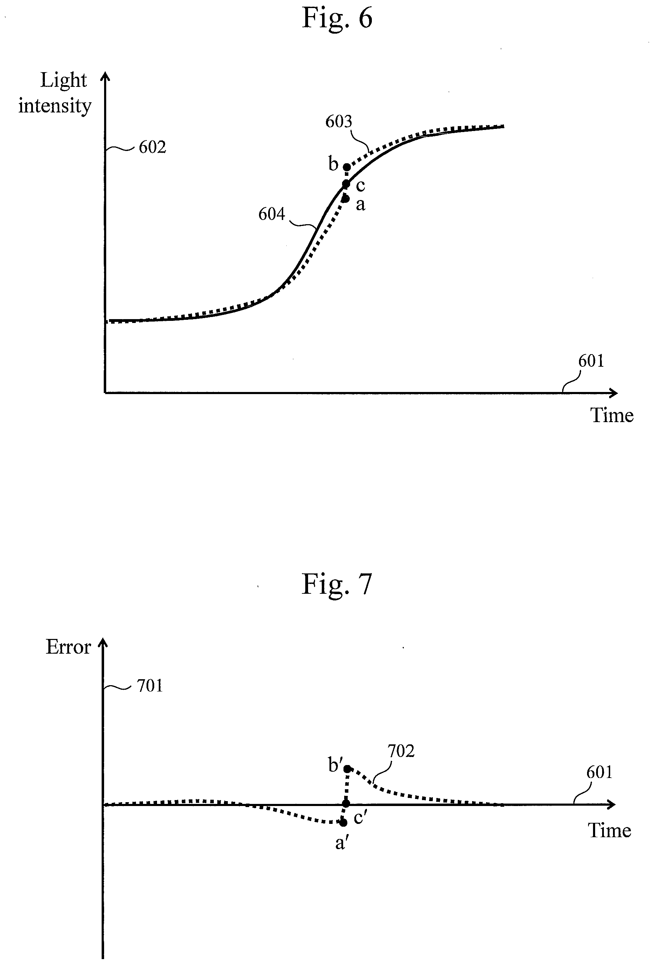

Hereinafter, processes of classifying the jump abnormality, the drift abnormality, the two-step reaction abnormality, and the noise abnormality will be described with reference to Figs. 6, 7, 8, 9, 10, 11, 12, and 13.

First, a process of classifying jump abnormality in step S301 will be described. In describing the process in step S301, an approximate curve calculation process in step S203 at the time of this abnormality classification, and a deviation feature information calculation process in step S204 will be described with reference to Figs. 6 and 7, respectively.

Fig. 6 illustrates a process image when the approximate curve calculation process in step S203 is applied to time-series light intensity data when including jump abnormality. A horizontal axis 601 indicates an elapse of time from reaction start, and a vertical axis 602 indicates the intensity of light. A point curve 603 indicates data points schematically indicating time-series light intensity data including jump abnormality. A solid curve 604 indicates a calculated approximate curve.

The point curve 603 includes jump abnormality (a portion indicated by a dotted line a-b) in which the amount of change in reaction is locally changed with respect to time during the reaction. If the approximate curve approximates to the time-series light intensity data having such an abnormality, a deviation between the time-series light intensity data and the approximate curve increases at a time at which the abnormality occurs and a time before and after that time. In this example, at a jump place, there is a point at which the approximate curve and the time-series light intensity data intersects (sign c).

Fig. 7 is a diagram obtained by calculating, in step S204, an error between the time-series light intensity data 603 and the approximate curve 604 illustrated in Fig. 6 as the deviation feature information of the approximate curve and the time-series light intensity data calculated in step S203 and plotting the error. A horizontal axis 601 indicates the same meaning as in Fig. 6. A vertical axis 701 is an axis indicating the error between the time-series light intensity data 603 and the approximate curve 604 in Fig. 6. A point curve 702 is a plot of an error value between the time-series light intensity data 603 and the approximate curve 604. Here, signs a', b', and c' on the point curve 702 correspond to the error values calculated from the data points at positions of signs a, b, and c on the point curve 603 in Fig. 6, respectively.

It can be seen from a comparison of the point curve 603 in Fig. 6 with the point curve 702 in Fig. 7 that a portion indicated by a dotted line a-c-b that is an area in which the abnormality occurs in the point curve 603 is expressed in a shape in which the amount of change in a portion indicated by a dotted line a' -c' -b' is greatly different from the other range and, specifically, a shape in which a value rapidly increases as a result of an error at a place of a point a' or a point b' in the point curve 702 increasing.

In step S301, a threshold value is set in the amount of change in the time-series error value indicated by the point curve 702 to detect a jump abnormality. The jump abnormality can be classified by extracting an area in which the amount of change is greatly changed in comparison with other portions, like a portion indicated by a dotted line a'-b'.

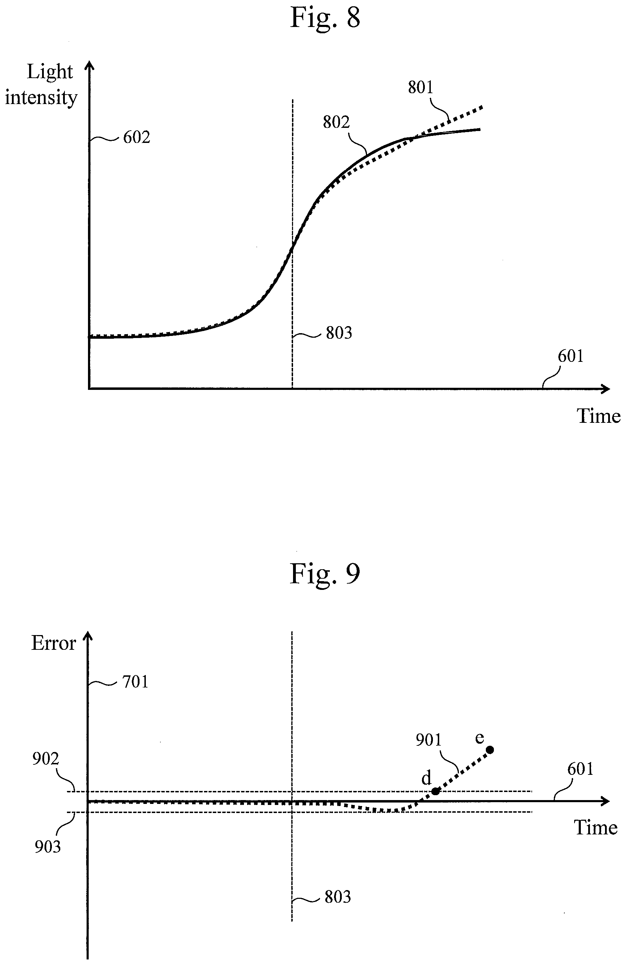

Next, the process of classifying the drift abnormality in steps S304 and S307 will be described. In describing the process of classifying the drift abnormality, the approximate curve calculation process in step S203 at the time of this abnormality classification and the deviation feature information calculation process in step S204 will be described with reference to Figs. 8 and 9, respectively.

Fig. 8 is a diagram illustrating a process image when the approximate curve calculation process in step S203 is applied to time-series light intensity data including drift abnormality. In addition, in Fig. 8, a tentative coagulation time calculated from the approximate curve in step S205 is also illustrated. A horizontal axis 601 and a vertical axis 602 indicate the same meaning as in Fig. 6. A point curve 801 indicates data points schematically indicating time-series light intensity data including the drift abnormality. A solid curve 802 indicates a calculated approximate curve. A dashed straight line 803 indicates a tentative coagulation time calculated from the approximate curve.

The point curve 801 includes the drift abnormality in which the intensity of light continues to change without a reaction rate not converging to a small value, in a second half of a coagulation reaction. If the approximate curve approximates to the time-series light intensity data having such an abnormality, a deviation between the time-series light intensity data and the approximate curve increases in a time range in which a drift occurs.

Fig. 9 is a diagram obtained by plotting a result of calculating, in step S204, an error between the time-series light intensity data 801 and the approximate curve 802 illustrated in Fig. 8 as the deviation feature information of the approximate curve and the time-series light intensity data calculated in step S203 and applying the abnormality occurrence range calculation process in step S302. In addition, in Fig. 9, a tentative coagulation time calculated from the approximate curve in step S205 is also illustrated. A horizontal axis 601 indicates the same meaning as in Fig. 6. A vertical axis 701 indicates the same meaning as in Fig. 7. A point curve 901 is a plot of an error value between the time-series light intensity data 801 and the approximate curve 802 in Fig. 8. A dashed straight line 902 and a dashed straight line 903 indicate threshold values set in order to calculate the abnormality occurrence range in step S302. A dotted line d-e indicates an abnormality occurrence range calculated in step S302. The dashed straight line 803 has the same meaning as in Fig. 8.

In the drift abnormality as shown in this example, it can be seen, from a comparison between the abnormality occurrence range indicated by the dotted line d-e with the tentative coagulation time indicated by the dashed straight line 803, that the abnormality occurrence range (d-e) is after the coagulation time 803. Accordingly, in step S304, the abnormality can be determined to frequently occur in the second half by determining a position of the abnormality occurrence range.

Further, in step S307, the drift abnormality can be determined by determining a feature amount of the time-series error data in the abnormality occurrence range. For example, using a slope of the dotted line d-e or a parameter obtained by regression of a primary equation as the feature amount, a degree of drift abnormality can be quantified and the drift abnormality can be determined.

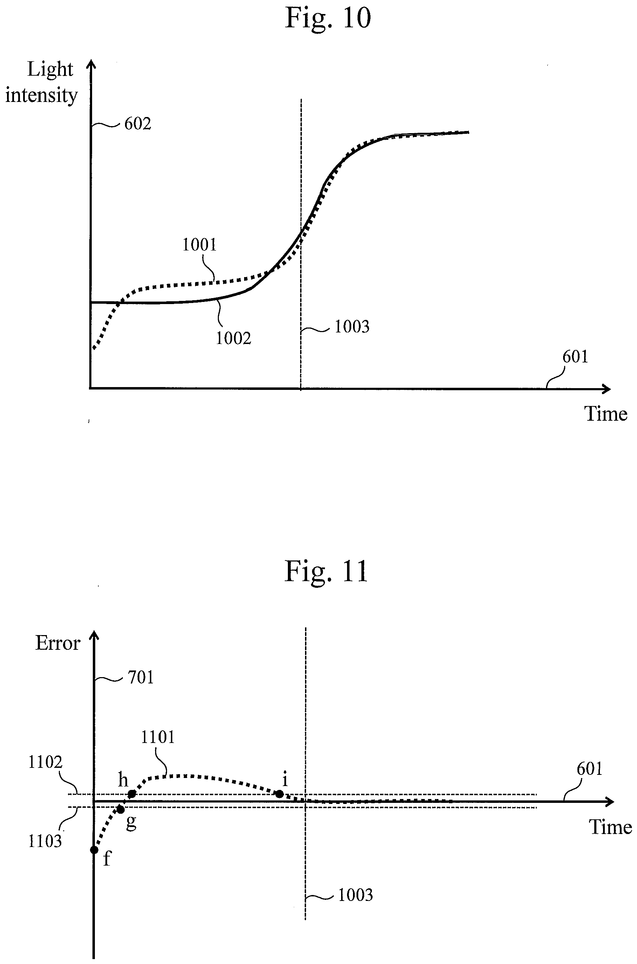

Next, the process of classifying the two-step reaction abnormality in steps S305 and S306 will be described. In describing the process of classifying the two-step reaction abnormality, the approximate curve calculation process in step S203 at the time of this abnormality classification, and the deviation feature information calculation process in step S204 will be described with reference to Figs. 10 and 11, respectively.

Fig. 10 is a diagram illustrating a process image when the approximate curve calculation process in step S203 is applied to time-series light intensity data having the two-step reaction abnormality. In addition, in Fig. 10, a tentative coagulation time calculated from the approximate curve in step S205 is also illustrated. A horizontal axis 601 and a vertical axis 602 have the same meaning as in Fig. 6. A point curve 1001 indicates data points schematically indicating the time-series light intensity data including the two-step reaction abnormality. A solid curve 1002 indicates a calculated approximate curve. A dashed straight line 1003 indicates a tentative coagulation time calculated from the approximate curve.

The point curve 1001 includes a two-step reaction abnormality in which a reaction rate once decreases in a first half of the coagulation reaction and then increases again. If the approximate curve approximates to the time-series light intensity data having such an abnormality, the deviation between the time-series light intensity data and the approximate curve increases in a time range in which the two-step reaction abnormality occurs.

Fig. 11 is a diagram obtained by plotting a result of calculating, in step S204, an error between the time-series light intensity data 1001 and the approximate curve 1002 illustrated in Fig. 10 as the deviation feature information of the approximate curve and the time-series light intensity data calculated in step S203 and applying the abnormality occurrence range calculation process in step S302. In addition, in Fig. 11, a tentative coagulation time calculated from the approximate curve in step S205 is also illustrated. A horizontal axis 601 indicates the same meaning as in Fig. 6. A vertical axis 701 indicates the same meaning as in Fig. 7. A point curve 1101 is a plot of an error value between the time-series light intensity data 1001 and the approximate curve 1002. A dashed straight line 1102 and a dashed straight line 1103 indicate threshold values set in order to calculate the abnormality occurrence range in step S302. A dotted line f-g and a dotted line h-i indicate abnormality occurrence ranges calculated in step S302. The dashed straight line 1003 has the same meaning as in Fig. 10.

In the two-step reaction abnormality as shown in this example, it can be seen, from a comparison between the abnormality occurrence ranges indicated by the dotted line f-g and the dotted line h-i with the tentative coagulation time indicated by the dashed straight line 1003, that the abnormality occurrence range is before the coagulation time. Accordingly, in step S305, the abnormality can be determined to frequently occur in the first half by determining a position of the abnormality occurrence range.

Further, in step S306, the abnormality can be determined to be the two-step reaction abnormality by determining the feature amount of the time-series error data in the abnormality occurrence range. For example, using a slope of the dotted line f-g or a sum of errors of the dotted line h-i as the feature amount, a degree of two-step reaction abnormality can be quantified and the abnormality can be determined to be the two-step reaction abnormality. In addition, a percentage (ratio) between the abnormality occurrence ranges, of total values of the errors of the respective abnormality occurrence ranges may also be calculated as the feature amount.

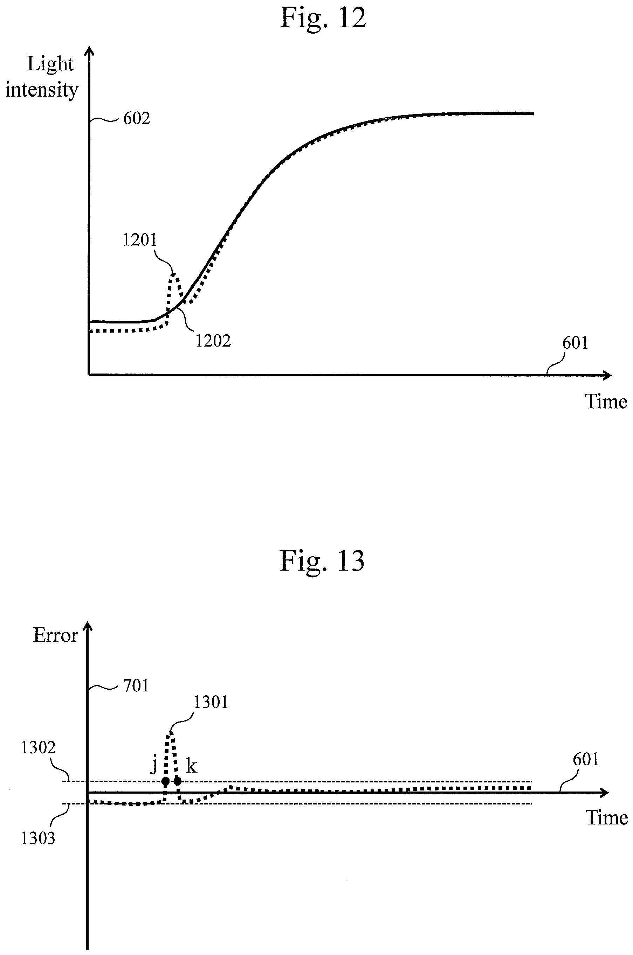

Next, a process of classifying the noise abnormality as the other abnormality in step S310 will be described. In describing the process of classifying the noise abnormality, the approximate curve calculation process in step S203 at the time of this abnormality classification, and the deviation feature information calculation process in step S204 will be described with reference to Figs. 12 and 13, respectively.

Fig. 12 is a diagram illustrating a process image when the approximate curve calculation process in step S203 is applied to a reaction curve having a noise abnormality. A horizontal axis 601 and a vertical axis 602 indicate the same meaning as in Fig. 6. A point curve 1201 indicates data points schematically indicating the time-series light intensity data including a noisy abnormality. A solid curve 1202 indicates a calculated approximate curve.

A point curve 1201 includes a noise abnormality in which the intensity of light fluctuates in a first half of the coagulation reaction. If the approximate curve approximates to the time-series light intensity data having such an abnormality, a deviation between the time-series light intensity data and the approximate curve increases in the time range in which a noise abnormality occurs.

Fig. 13 is a diagram obtained by plotting a result of calculating, in step S204, an error between the time-series light intensity data 1201 and the approximate curve 1202 illustrated in Fig. 12 as the deviation feature information of the approximate curve and the time-series light intensity data calculated in step S203 and applying the abnormality occurrence range calculation process in step S302. A horizontal axis 601 indicates the same meaning as in Fig. 6. A vertical axis 701 indicates the same meaning as in Fig. 7. A point curve 1301 is a plot of an error value between the time-series light intensity data 1201 and the approximate curve 1202. A dashed straight line 1302 and a dashed straight line 1303 indicate threshold values set in order to calculate the abnormality occurrence range in step S302. A dotted line j-k indicates an abnormality occurrence range calculated in step S302.

In the noise abnormality as shown in this example, the shape tends to be sharp in the abnormality occurrence range indicated by the dotted line j-k. Therefore, the noise abnormality can be determined by paying attention to the feature amount indicating the shape. For example, the determination may be performed using a ratio of the abnormality occurrence range (a duration on the axis of a dotted line j-k) and an error maximum value (a value of a peak in the range of the dotted line j-k in Fig. 13) of the abnormality occurrence range. In an example of Figs. 12 and 13, an example of noise in which an error value protrudes in a short range on the time axis is shown, but other abnormalities such as a noise abnormality are not limited thereto. For example, an abnormality in which the abnormality occurrence range is a wider range on the time axis, that is, noise having a large error with respect to the approximate curve for a long time may be a target.

Hereinafter, a modification example of the first embodiment will be described. Although the example in which the control unit 121 performs the processes shown in Figs. 2 and 3 has been shown in the first embodiment described above, other units of the device may perform the processes. For example, the processes of Figs. 2 and 3 can also be executed as software within the operation computer 118.

Although the example in which the approximation function, processing data during the process, and the result of the process are stored in the storage unit 119 in the first embodiment described above, the approximation function, process data during the process, and the result of the process may also be stored in another unit of the device. For example, a storage unit inside the operation computer 118 may also be used.

Although the example in which Equation (1) is used as the approximation function in step S201 has been described in the first embodiment described above, an approximation function that can be used in the present invention is not limited to Equation (1), and a growth curve type growth function may be widely used. The growth function described here is a function characterized by a shape in which a change amount with respect to time decreases in an initial stage, gradually increases, and decreases again in a late stage. For example, an example of a growth function includes a function having a first area in which the amount of change in the measured value with respect to time gradually increases, and a second area in which the amount of change in the measured value with respect to time at a time after the first area is smaller than that in the first area. Examples of a specific growth curve type function may include a logistic function, a Gompertz function, and a Hill function. Further, for example, a function in which some of items of the growth function is subjected to exponentiation, the growth function is multiplied by a non-linear equation regarding time, the non-linear equation regarding time is added to the growth function, or an input value of the growth function is subjected to non-linear conversion using a non-linear equation in advance may be used. As the non-linear equation, for example, a polynomial equation may be used.

Although the example in which the control unit 121 uses the taken time-series light intensity data as it is in step S202 has been described in the first embodiment described above, a smoothing filter may be applied to the light intensity value of the taken time-series light intensity data, and time-series light intensity data after smoothing may be used. As the smoothing filter, a known smoothing scheme such as a moving average filter or a central value filter may be widely applied.

In the first embodiment described above, the example in which the control unit 121 calculates the value of the parameter in the approximation function so that the difference between the approximate curve and the time-series light intensity data becomes as small as possible in step S203 has been described, but a method of calculating the value of the parameter is not limited thereto, and a value of the parameter in the approximation function may be calculated so that an index other than the difference between the approximate curve and the time-series light intensity data may be decreased or increased, or to be close to a specific value. For example, only a portion of the data time-series light intensity data may be selected and the value of the parameter may be calculated so that the difference between the data and the approximate curve becomes as small as possible. Further, a different weight may be defined for each data point in the time-series light intensity data, and the value of the parameter may be calculated so that an index obtained by multiplying a difference between each data point and the approximate curve by each weight becomes as small as possible. Further, a deviation state from a target value of each parameter may be used as an index.

Although the example in which the control unit 121 calculates error data in time-series (time-series error data) between the time-series light intensity data and the approximate curve is calculated as the deviation feature information in step S204 has been described in the first embodiment described above, other information may be used. For example, time-series data of an absolute value of the error may be used or a sum of errors may be used. Further, a determination coefficient indicating a degree of approximation may be used. Further, a vector obtained by combining a plurality of such indices may be used.

Although the example in which the control unit 121 deals with the tentative coagulation time calculated on the basis of the approximate curve in step S205 as the feature amount has been described in the first embodiment described above, other feature amounts may be used. For example, a parameter in the approximation function corresponding to the acquired approximate curve, a reaction end time calculated on the basis of the approximate curve, or the like may be used. Further, a maximum value, a data length, a coagulation time, a reaction end time, or the like may be calculated from the time-series light intensity data that is raw data and may be used.

The example in which the control unit 121 calculates the number of data points having the error equal to or greater than the threshold value and compares the calculated number of data points with the threshold value of the number of abnormality data points to determine the abnormality in step S206 has been described in the first embodiment described above, but the abnormality determination method is not limited thereto. For example, the threshold value may be set for the absolute value of the error and a threshold determination may be performed, or different threshold values may be set for respective positive and negative values of the error and a threshold determination may be performed. Accordingly, for example, a modification example in which the abnormality is determined on the basis of only the data points in an upper portion of the approximate curve may also be realized. Further, a threshold value may be set for a value such as a square error sum of the time-series light intensity data and the approximate curve, or a determination coefficient and the determination may be performed.

In the first embodiment described above, the control unit 121 performs the abnormality classification using the decision tree illustrated in Fig. 3 in step S208, but the decision tree for use in the abnormality classification is not limited to that illustrated in Fig. 3. For example, the classification may be performed using a plurality of decision trees for evaluating independently the classification of respective abnormalities, or the classification may be performed using different decision trees.

In the first embodiment described above, the example in which the control unit 121 repeatedly applies a determination process to the deviation feature information calculated in step S204 on the basis of the decision tree stored in the storage unit 119 in advance to classify the type of abnormality included in the time-series light intensity data in step S208, but the abnormality classifying method is not limited thereto. For example, the storage unit 119 may store any-dimensional space definition, and any number of vectors associated with each abnormality code, and the control unit 121 may map the deviation feature information calculated in step S204 to the space through linear mapping or nonlinear mapping, and then, determine the abnormality code using an existing classification scheme. As the classification scheme, for example, a known scheme such as a re-neighborhood search method, a k neighborhood method, or a support vector classifier may be used. Further, for example, the storage unit 119 may store an evaluation function of taking the deviation feature information or any-dimensional vector obtained by converting the deviation feature information through linear mapping or nonlinear mapping as an argument, and returning a value corresponding to each abnormality code, and the control unit 121 may classify the deviation feature information calculated in step S204 or the vector obtained by linearly mapping or nonlinearly mapping the deviation feature information, using the evaluation function. As the evaluation function, a known scheme such as a neural network may be used.

That is, as a modification example of the classification process, the storage unit 119 may store any one of (1) at least one any-dimensional vector associated with the type of abnormality, (2) an identification boundary in at least one any-dimensional space associated with the type of abnormality, (3) a partial space in at least one any-dimensional space associated with the type of abnormality, and (4) an evaluation function having any-dimensional vector as an argument and an value associated with the type of abnormality as an output value, and the control unit 121 may convert the deviation feature information into any-dimensional vector feature information through linear or non-linear mapping and classify the type of abnormality on the basis of the any-dimensional vector, the identification boundary, the partial space, or the output value of the evaluation function. It is possible to perform the abnormality classification by applying the feature amount of the data of the measurement value to any abnormal pattern using a scheme other than decision tree.

In the first embodiment described above, the example in which the control unit 121 compares the value of each data point of the time-series error data with a preset threshold value, determines the data point having a value equal to or greater than the threshold value to be an abnormal data point, and then calculates the abnormality occurrence range in step S302 has been described, but a method of calculating the abnormality occurrence range is not limited thereto. For example, a threshold value may be set for an absolute value of the error, a data point subjected to a threshold determination may be determined to be an abnormal data point, and the abnormality occurrence range may be calculated, or different threshold values may be set for respective positive and negative values of the error, a data point subjected to a threshold determination may be determined to be the abnormal data point, and the abnormality occurrence range may be calculated.

The example in which the control unit 121 calculates the feature amount regarding the shape of the time-series error data of the abnormality occurrence range in step S303 has been described in the first embodiment described above, but this feature amount may be calculated using all of items of time-series error data.

Although the example in which the control unit 121 performs the process of determining the abnormality in step S206 and the process of classifying the abnormality in step S208 has been described in the first embodiment described above, the processes may be collectively performed in the same step. For example, the storage unit 119 may store a code corresponding to a normal reaction in advance, and the control unit 121 may pass though steps S206 and S207 without performing any process, and simultaneously perform the abnormality determination when performing the abnormality classification and read the code corresponding to the normal reaction for the sample in which the abnormality does not occur in step S208.

In the first embodiment described above, the example in which the control unit 121 reads the abnormality code stored in the storage unit 119 on the basis of the type of classified abnormality in step S208 and outputs content thereof in step S209 has been described, but the present invention is not limited thereto. Information other than the above may be calculated in step S208 and the information may be output in step S209. For example, an index indicating the certainty of the abnormality classification may also be calculated in step S208 and output in step S209. The index indicating the certainty of the abnormality classification may be calculated, for example, on the basis of a relationship between the feature amount and the value of the threshold value. In a case in which the value of the feature amount greatly exceeds the threshold value, the certainty of the abnormality classification can be determined to high, and in a case in which the value of the feature amount is near the threshold value, the certainty of the abnormality classification can be determined to low. The index may be calculated and output as qualitative information such as high/low certainty, or a quantitative value, such as a value of a percentage indicating the certainty, may be calculated and output on the basis of the amount exceeding the threshold value.

Although the coagulation reaction curve of the time-series light intensity data, the approximate curve, and the abnormality occurrence range have been output in the same figure on the output screen in step S209 in the first embodiment described above, the coagulation reaction curve, the approximate curve, and the abnormality occurrence range may be output in separate figures. Further, for example, a color may be changed or the shape of the data points may be changed for output so that the abnormality occurrence range is distinguished. Further, content of the output is not limited to the example of Fig. 14. An output unit such as the operation computer 118 or the printer 124 may output at least one of (1) the time-series light intensity data output using a first axis as time and a second axis as a light intensity value, (2) the approximate curve output using a first axis as time and a second axis as a light intensity value, (3) the deviation feature information, (4) a result of analysis (for example, a blood coagulation time), (5) an equation of the approximation function, (6) a result of the abnormality detection (information on whether the abnormality has been detected), (7) a result of the abnormality classification (information such as abnormality codes), and (8) information on a process of coping with the abnormality (information on a method of coping with each abnormality).

In the first embodiment described above, abnormalities such as the two-step reaction, the drift reaction, jump reaction, and the noise have been described, but the present invention is not limited to the abnormally. The detected and classified abnormality may include, for example, at least one of (1) an abnormality in which the amount of a change in the measured value in the time-series data (for example, the light intensity value) rapidly increases or decreases, (2) an abnormality in which the amount of a change in the measured value in the time-series data once increases and decreases, and then, increases again, and (3) an abnormality in which the amount of a change in the measured value in the time-series data remains at a certain value or in a predetermined range (that is, abnormality in which the measured value does not converge).

As described above, in the first embodiment of the present invention, it is possible to detect and classify abnormalities such as the two-step reaction, the drift reaction, the jump reaction, and the noise unique to the blood coagulation reaction by calculating the approximate curve of the time-series light intensity data, calculating the deviation feature information between the time-series light intensity data and the approximate curve, and analyzing the deviation feature information, and to present information on the abnormalities.

[Second Embodiment]

In a second embodiment of the present invention, an operation example different from that in the first embodiment in a scheme of classifying an abnormality will be described. Since a configuration of an automatic analytical apparatus is the same as that in the first embodiment, a difference between the operations of the control unit 121 will be described.

In the second embodiment, the control unit 121 normalizes the deviation feature information using information acquired from the time-series light intensity data acquired for each sample or the approximate curve of the time-series light intensity data. In general, a reaction curve acquired in the coagulation test has characteristics in that the amount of a change in the reaction rate or the intensity of light is different according to a concentration of a target substance contained in the sample. In this embodiment, an automatic analytical device capable of accurately classifying abnormality even in a case in which there are samples having a difference between reaction curve shapes by normalizing the deviation feature information acquired from the time-series light intensity data and the approximate curve.

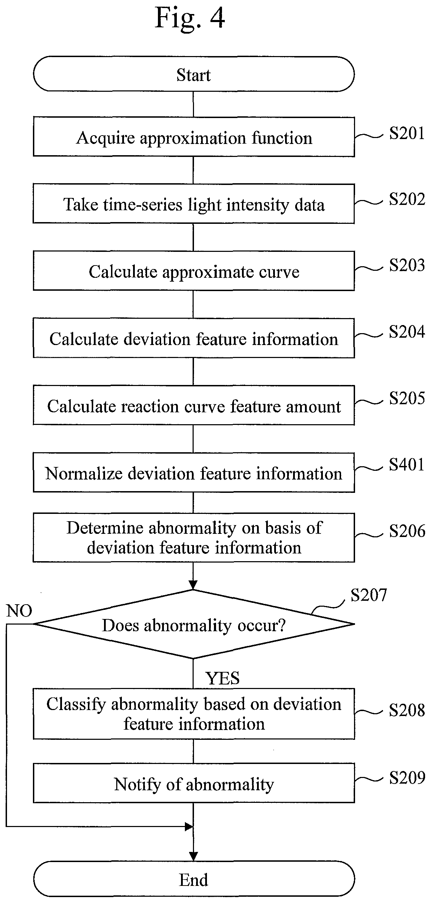

Fig. 4 is a diagram illustrating a process flow in which the control unit 121 performs abnormality detection and classification in the second embodiment. In the process flow illustrated in Fig. 4, the same steps as those described in Fig. 2 in the first embodiment are denoted with the same step numbers. That is, since the process from step S201 to step S205 and the process from step S206 to step S209 are the same as in Fig. 2, the description thereof is omitted. In the second embodiment, step S401 is introduced between step S205 and step S206. In step S401, the control unit 121 normalizes the deviation feature information calculated in step S204. The normalization is performed using the reaction curve feature amount calculated in step S205.

Hereinafter, an example of the normalization method will be described. A first example of the normalization method is normalization of a time axis direction for the deviation feature information having time information. An example of the deviation feature information having the time information includes time-series error data obtained by calculating an error between the time-series light intensity data and the approximate curve for each time-series light intensity data point. Time information in this case is a measurement time of data that each data point included in the time-series error data has. An example of the normalization method includes a method of dividing the time information that the deviation feature information has, using the tentative coagulation time calculated from the approximate curve in step S205 and converting the time information to be a percentage with respect to the tentative coagulation time.

A second example of the normalization method is normalization in a light intensity axis direction for the deviation feature information having light intensity information. An example of the deviation feature information having light intensity information includes time-series error data obtained by calculating an error between the time-series light intensity data and the approximate curve for each time-series light intensity data point. Light intensity information in this case is an error value that each data point included in the time-series error data has. An example of the normalization method includes a method of dividing the light intensity information that the deviation feature information has, using a maximum light intensity value calculated from the approximate curve in step S205 and converting the light intensity information to be a percentage with respect to the maximum light intensity value.

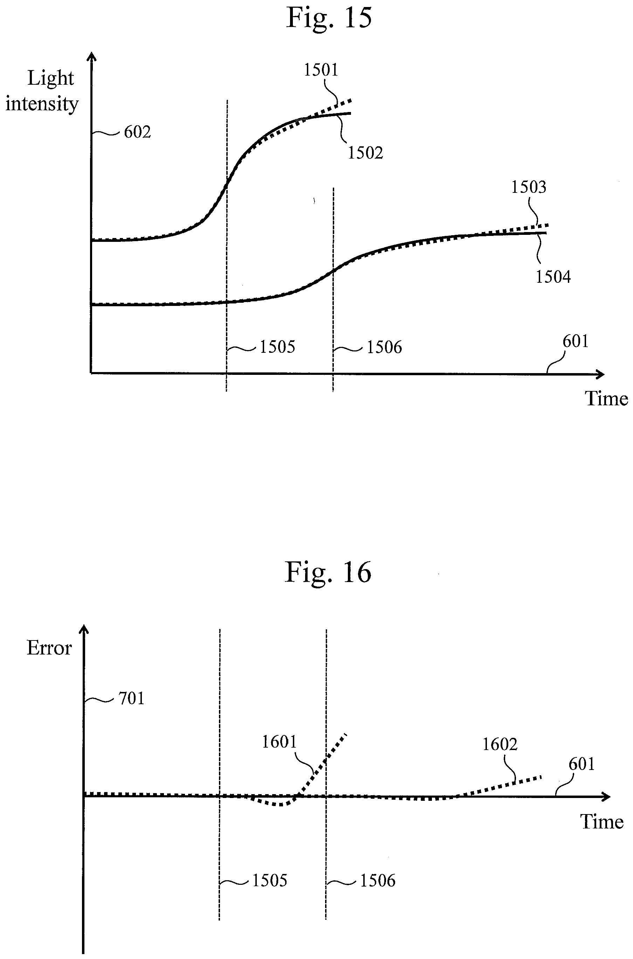

Hereinafter, an example and an effect of the normalization of the deviation feature information in the second embodiment will be described with reference to Figs. 15, 16, 17, and 18. In this description, an example in which the time-series error data is calculated as the deviation feature information in step S204, the tentative coagulation time and the light intensity value at the time of reaction end are calculated as reaction curve feature amounts from the approximate curve in step S205, and the normalization in the time axis direction and the normalization in the light intensity axis direction are continuously performed in step S401 will be described.

First, a case in which, when the reaction curve shape is different for each sample, the deviation feature information is affected by the difference is shown using Figs. 15 and 16. Fig. 15 is an image diagram simultaneously illustrating the time-series light intensity data acquired in step S202 from two different samples and the approximate curve acquired in step S203. Any of the time-series light intensity data also has the drift abnormality in which a reaction rate is not zero in a second half of the reaction. A horizontal axis 601 and a vertical axis 602 indicate the same meanings as in Fig. 6. A point curve 1501 indicates data points schematically illustrating time-series light intensity data including the drift abnormality acquired from a first sample. A solid curve 1502 indicates an approximate curve calculated from the time-series light intensity data indicated by the point curve 1501. A point curve 1503 indicates data points schematically indicating the time-series light intensity data including the drift abnormality acquired from a second sample. A solid curve 1504 indicates an approximate curve calculated from the time-series light intensity data indicated by the point curve 1503.

Fig. 16 is an image diagram simultaneously illustrating both of a result of calculating, in step S206, the time-series error data between the time-series light intensity data 1501 and the approximate curve 1502 illustrated in Fig. 15 as the deviation feature information of the approximate curve and the time-series light intensity data calculated in step S203 and the time-series error data between the time-series light intensity data 1503 and the approximate curve 1504 in step S206. A horizontal axis 601 indicates the same meaning as in Fig. 6. A vertical axis 701 indicates the same meaning as in Fig. 7. A point curve 1601 is a plot of an error value between the time-series light intensity data 1501 and the approximate curve 1502. A point curve 1602 is a plot of an error value between the time-series light intensity data 1503 and the approximate curve 1504.

It can be seen from Figs. 15 and 16 that both of the time-series light intensity data 1501 and the time-series light intensity data 1503 have the same drift abnormality, but the deviation feature information 1601 and the deviation feature information 1602 calculated from the time-series light intensity data 1501 and the time-series light intensity data 1503 have different shapes since scales in the time axis direction and the light intensity axis direction are different.

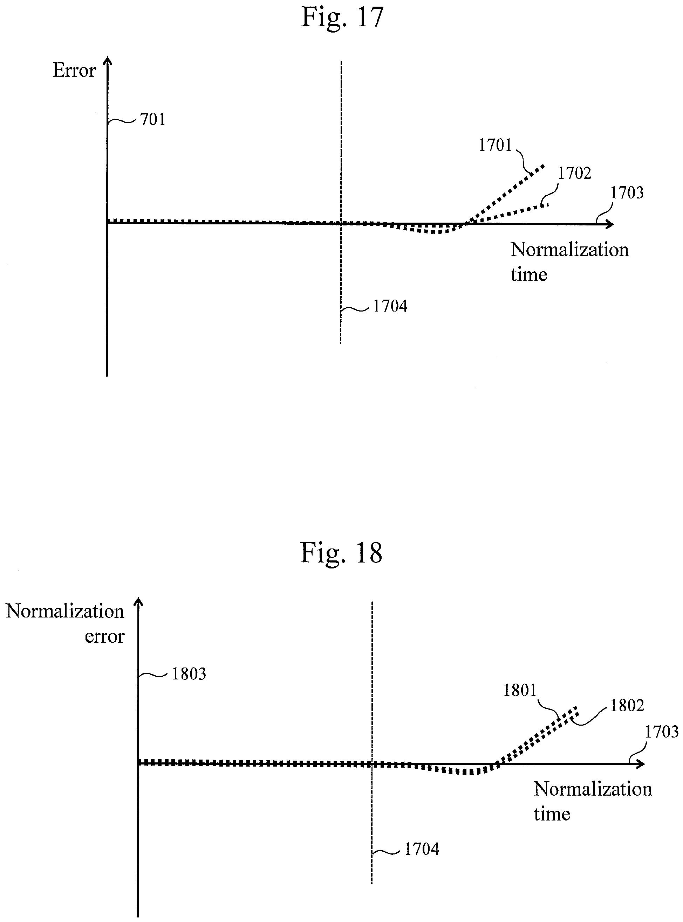

Next, a case in which normalization is applied will be described with reference to Figs. 17 and 18. Fig. 17 is an image diagram illustrating a result of performing normalization in the time axis direction on the time-series error data illustrated in Fig. 16. Here, an example in which the normalization is performed using the tentative coagulation time calculated for each sample is illustrated. Specifically, an example in which an acquisition time of each data point of the time-series error data is expressed as a percentage to the tentative coagulation time by dividing the acquisition time by the tentative coagulation time is illustrated. A point curve 1701 indicates time-series error data obtained by normalizing the time-series error data indicated by the point curve 1601 using the tentative coagulation time indicated by a dashed straight line 1505.

The point curve 1702 indicates time-series error data obtained by normalizing the time-series error data indicated by the point curve 1602 using the tentative coagulation time indicated by a dashed straight line 1506. A horizontal axis 1703 is an axis indicating an elapsed time from reaction start as a percentage with respect to the tentative coagulation time of each sample. A dashed straight line 1704 indicates a line after the tentative coagulation time calculated from the approximate curve 1502 and the approximate curve 1504 is subjected to a normalization process. A vertical axis 701 indicates the same meaning as in Fig. 7.

Fig. 18 is an image diagram illustrating a result of further performing the normalization in the light intensity axis direction on the time-series error data on which the normalization in the time axis direction has been performed, which is illustrated in Fig. 17. Here, an example in which the normalization is performed using light intensity values at the time of reaction end calculated from respective samples is shown. Specifically, an example in which an error value of each data point in the time-series error data is expressed as a percentage with respect to the light intensity value at the time of reaction end by being divided by the light intensity value at the time of reaction end. A point curve 1801 is a time-series error data obtained by normalizing the time-series error data indicated by a point curve 1701 using a light intensity value at the time of reaction end calculated from the approximate curve 1502. A point curve 1802 is a time-series error data obtained by normalizing the time-series error data indicated by a point curve 1702 using a light intensity value at the time of reaction end calculated from the approximate curve 1504. A vertical axis 1803 is an axis indicating an error between the time-series light intensity data and the approximate curve as a percentage to the light intensity value at the time of reaction end. A horizontal axis 1703 and a dashed straight line 1704 indicate the same meaning as in Fig. 17.

It can be seen that the point curve 1801 and the point curve 1802 are acquired as similar shapes as a result of a difference between scales in the time axis direction and the light intensity axis direction being relaxed, by performing the normalization, as illustrated in Fig. 18. The feature amount, the threshold value, and the like to be handled can be handled in a unified framework even in a case in which samples in different concentration zones are targets in the abnormality determination based on the deviation feature information in step S206 and the abnormality classification based on the deviation feature information in step S208 by performing the normalization, and it is possible to accurately perform the abnormality detection and the abnormality classification.

Hereinafter, a modification example of the second embodiment will be described. In the second embodiment described above, the example in which the normalization in the time axis direction is performed using the tentative coagulation time calculated from the approximate curve in step S401 has been described, but the value used for normalization is not limited thereto. For example, a feature amount that can be calculated from the approximate curve, such as the reaction end time or the parameter calculated from the approximate curve, may be widely used. Further, a feature amount that can be calculated from the time-series light intensity data, such as the coagulation time or the reaction end time calculated from the time-series light intensity data may also be used widely.

The example in which the normalization in the light intensity axis direction is performed using the light intensity value at the time of reaction end calculated from the approximate curve in step S401 has been described in the second embodiment described above, but the value used for normalization is not limited thereto. For example, the feature amount that can be calculated from the approximate curve, such as a parameter calculated from the approximate curve, may be widely used. Further, a feature amount that can be calculated from the time-series light intensity data, such as a maximum value, an average value, or a median value of the light intensity calculated from the time-series light intensity data, may be widely used.

In the second embodiment described above, the example in which the normalization is performed by calculating a percentage with respect to a specific value in step S401 has been described, but the normalization method is not limited thereto. For example, any linear transformation or nonlinear transformation such as a logarithmic transformation with a specific value as a bottom may be used.

In the second embodiment described above, the example in which the normalization in the time axis direction and the light intensity axis direction is performed in step S401 has been described, but a target of the normalization is not limited thereto. For example, in a case in which information based on the square error is calculated as the deviation feature information, the feature amount regarding the square error can be calculated from the time-series light intensity data and the approximate curve as the feature amount of the reaction curve, and the normalization may be performed using the feature amount.

As described above, in the automatic analytical apparatus according to the second embodiment, it is possible to accurately classify the abnormality in a case in which there are samples having a difference in the reaction curve shapes of the respective samples by normalizing the deviation feature information using the information acquired from the time-series light intensity data acquired for each sample or the approximate curve of the time-series light intensity data.

[Third Embodiment]

In a third embodiment of the present invention, a different operation example from those in the first and second embodiments in the scheme of classifying an abnormality will be described. Since the configuration of the automatic analytical apparatus is the same as those of the first embodiment and the second embodiment, a difference between the operations of the control unit 121 will be mainly described.

In a third embodiment, the control unit 121 repeatedly performs a reaction end determination process of determining whether the coagulation reaction has ended, and an abnormality determination and abnormality classification process. Conventionally, a worker was required to perform a determination as to whether an abnormality has occurred in a reaction, after the measurement has ended. However, if the worker can detect the occurrence of the abnormality and recognize the type of abnormality during the measurement, the worker can rapidly perform next countermeasure such as re-test, and efficiency of test work can be realized. Therefore, in the third embodiment, by repeatedly performing the reaction end determination and the abnormality determination and classification, the abnormality of the reaction is detected during the measurement, and the type of abnormality is classified and presented to the worker. Accordingly, the worker can recognize the occurrence and the type of abnormality from the reaction during the measurement.

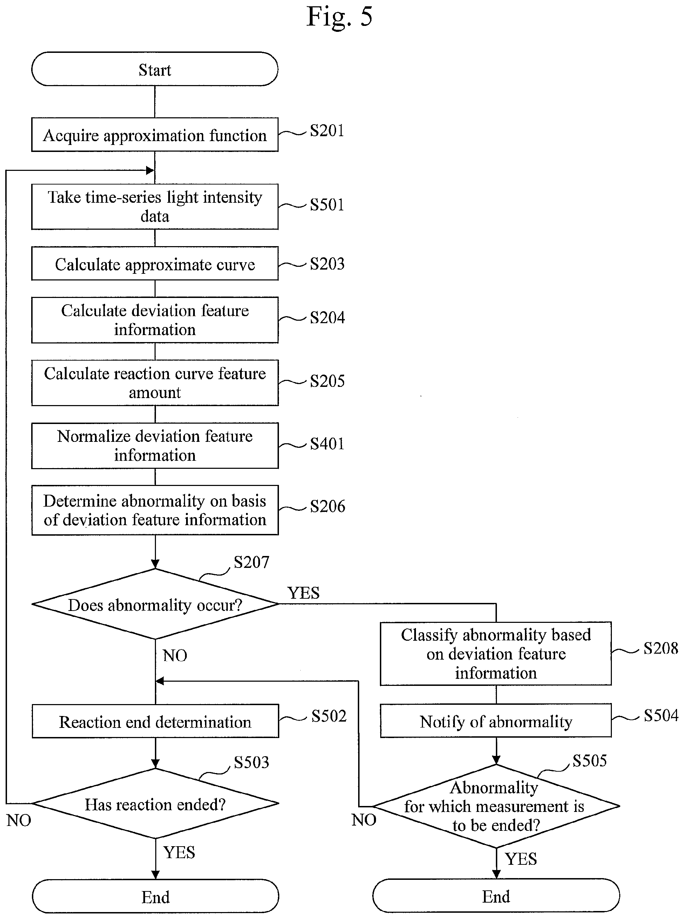

Fig. 5 is a diagram illustrating a process flow in which the control unit 121 performs the abnormality detection and classification in the third embodiment. In the process flow illustrated in Fig. 5, the same steps as those described in Fig. 2 in the first embodiment and those described in Fig. 4 in the second embodiment are denoted with the same step numbers . That is, since the processing of step S201 and the process from step S203 to step S208 are the same as in Fig. 2, a description thereof is omitted. Since the process in step S401 is the same as in Fig. 4, description thereof is omitted.

In this embodiment, step S501 is introduced instead of step S202 between step S201 and step S203. Further, step S502 and step S503 branched from step S207 are introduced. Further, step S504 and step S505 are introduced instead of step S209.

First, in step S501, the control unit 121 takes, as the time-series light intensity data, light/current conversion data detected by the detector 116 in a certain period, via the A/D converter 122 and the interface 123. If a predetermined number of pieces of data are taken, the process proceeds to step S203. Step S501 may be performed two or more times according to a determination result of step S503 to be described below. In this case, a determination as to whether a predetermined number of pieces of data have been taken may be performed using the number of data points newly taken from the start of step S501, or may be performed using a total number of data points taken in step S501 heretofore after the process starts.

In step S502, an end of the coagulation reaction is determined using the time-series light intensity data taken in step S501 and the reaction curve feature amount calculated in step S205, and the process proceeds to step S503. For an end determination of the coagulation reaction, a generally well known method may be used. For example, it can be determined that the coagulation reaction has completed using, for example, a scheme of setting a threshold value for a measurement time of the time-series light intensity data, setting a threshold value for the amount of a change in recent light intensity value, or setting a threshold value for a parameter of the approximate curve.

In a case in which a determination result in step S502 indicates the end of the coagulation reaction in step S503, the process ends. Otherwise, the process proceeds to step S501.

In step S504, the information on the abnormality stored in the storage unit 119 is read using the abnormality code calculated in step S208 as a key, and output to the operation computer 118 or the printer 124. For the information to be output, the information described in the first embodiment may be used. In order to report an output result to a worker performing test work, for example, warning sound or a warning screen informing the worker of the abnormality on a screen may be output.

In step S505, it is determined whether the measurement is to be ended according to the type of abnormality classified in step S208, and in a case in which the measurement is not to be ended, the process proceeds to a reaction end determination step S502. For example, an abnormality code, and information (for example, a flag) indicating whether the measurement is to be ended are stored in association with each other in the storage unit 119, and the control unit 121 may determine whether the measurement is to be ended on the basis of this information. In step S504, when the abnormality is reported to a user, the user may be allowed to select whether the measurement is to be ended or to continue.

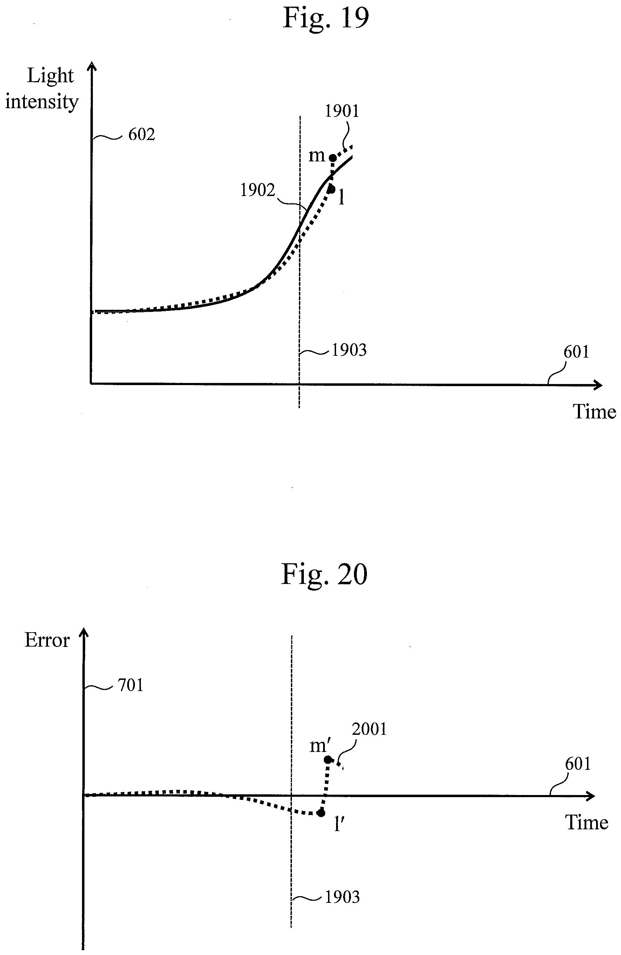

Hereinafter, effects of the abnormality detection and classification in the third embodiment will be described with reference to Figs. 19 and 20. In this description, an example in which the time-series error data is calculated as the deviation feature information in step S204 will be described.

Fig. 19 is a diagram illustrating a process image when approximate curve processing in step S203 is applied to the time-series light intensity data taken in time-series light intensity data acquisition step S501. In addition, in Fig. 19, the tentative coagulation time calculated from the approximate curve in step S205 is also illustrated. Here, in the time-series light intensity data, a jump abnormality (a portion indicated by a dotted line l-m) in which the intensity of light suddenly changes occurs. A horizontal axis 601 and a vertical axis 602 indicate the same meaning as in Fig. 6. A point curve 1901 indicates data points schematically indicating the time-series light intensity data including the jump abnormality. A solid curve 1902 indicates a calculated approximate curve. A dashed straight line 1903 indicates the tentative coagulation time calculated in step S205.

Fig. 20 is a diagram obtained by calculating, in step S204, an error between the time-series light intensity data 1901 and the approximate curve 1902 illustrated in Fig. 19 as the deviation feature information of the approximate curve and the time-series light intensity data calculated in step S203 and plotting the error. A horizontal axis 601 indicates the same meaning as in Fig. 6. A vertical axis 701 indicates the same meaning as in Fig. 7. A dashed straight line 1903 indicates the same meaning as in Fig. 19.

It can be seen that the time-series light intensity data 1901 is taken up to only the data point at which the reaction still continues, and the coagulation reaction does not end, but an abnormality (a portion indicated by a dotted line l'-m') is expressed as an area having a large change amount in the deviation feature information of a point curve 2001. Thus, it is possible to perform the abnormality determination in step S206 and the abnormality classification in step S208 from the data during the reaction using the deviation feature information between the time-series light intensity data and the approximate curve.

Further, since information such as the coagulation time can also be acquired from the approximate curve or the time-series light intensity data during the reaction, the normalization process in step S401 can also be applied to the curve during the reaction, and accurate classification can be performed on a reaction curve derived from a sample having a different concentration zone.

Hereinafter, a modification example of the third embodiment will be described. Although the example in which the normalization is performed in step S401 has been described in the third embodiment described above, the normalization process is not necessarily required, and may not be performed.

Although the example which the information on the abnormality stored in the storage unit 119 is read and output using the abnormality code calculated in step S208 as a key in step S504 has been described in the third embodiment described above, other information may be output. For example, an index indicating certainty of the abnormality classification calculated in step S208 may output after each abnormality determination and abnormality classification process is performed. Accordingly, a sign of the abnormality occurrence can be presented to a worker before the occurrence of the abnormality is determined.

As described above, in the automatic analytical apparatus according to the third embodiment, it is possible to detect the abnormality of the reaction during the measurement, classify the type of abnormality, and present a result thereof to the worker by repeatedly performing the reaction end determination, the abnormality determination, and the classification during the measurement.

The function, processing means, and the like of the control unit 121 described above, may be realized by hardware, for example, by designing some or all thereof using an integrated circuit. Further, the function, processing means, and the like of the control unit 121 described above, may be realized by software by a processor interpreting and executing a program realizing the respective functions. Information such as a program, a table, or file realizing each function can be stored in a storage device such as a memory, a hard disk, or a solid state drive (SSD), or a storage medium such as an IC card, an SD card, or a DVD.

Further, control lines or information lines in the drawings considered to be required for description have been shown, and all of control lines or information lines on a product are not necessarily shown. All configurations may be connected to one another.

Reference sign list

101    sample dispensing unit
102    sample disk
103    sample container
104    reaction container
105    sample syringe pump
106    reagent dispensing unit
107    reagent disk
108    reagent container
109    reagent heating unit
110    reagent syringe pump
111    reaction container stock unit
112    reaction container transport unit
113    detection unit

114     reaction container installation unit
115     light source
116     detector
117     reaction container discardment unit
118     operation computer
119     storage unit
120     input unit
121     control unit
122     A/D converter
123     interface
124     printer

**Claims**

1.  An automatic analytical apparatus, comprising:

     a reaction container (104) for mixing a sample with a reagent to form a reaction solution;
     a measurement unit (115, 116) that is configured to irradiate a reaction solution in the reaction container with light and to measure a time-series of the intensity of transmitted light or scattered light;
     a control unit (121) that is configured to process the time-series light intensity data obtained through the measurement in the measurement unit;
     a storage unit (119) that is configured to store one or more approximation functions each approximating to a time-series change in the light intensity data; and
     an output unit (118, 124) that is configured to output a processing result of the control unit (121), wherein the output unit (118, 124) is configured to output information on a detected and classified abnormality;
     wherein
     the control unit (121) is configured to

          select any one of the approximation functions stored in the storage unit (119),
          calculate an approximate curve indicating a time-series change in the light intensity data using the selected approximation function,
          calculate deviation feature information based on deviation information between the light intensity data and the approximate curve, wherein deviation feature information is defined as information representing a degree of approximation of the approximate curve to the time-series light intensity data, and
          detect and classify an abnormality included in the light intensity data using
          time-series error data as the deviation feature information wherein the control unit (121) is configured to calculate the time-series error data indicating a time-series change in an error between the light intensity data and the approximate curve, said error being the difference between the light intensity calculated using the approximate curve and the time-series light intensity data;

     **characterized in that**,
     in order to determine whether an abnormality is included in the light intensity data, the control unit (121) is configured to:
     set a threshold-error-value, which is a threshold value of an error for the time-series error data, calculate the number of data points having an error equal to or greater than the threshold-error-value, and compare the calculated number of data points with a threshold value for the number of abnormality data points to determine if an abnormality is present; and
     in order to classify the abnormality, the control unit (121) is configured to:
     determine whether there is a sudden change in the time-series error data, wherein said sudden change is detected by setting a threshold value in the amount of change between an error of a certain data point and an error of a subsequent data point and if a sudden change is present, classify the detected abnormality as a jump abnormality and if a sudden change is not present:

          calculate a range in the time-series in which the abnormality occurs in the time-series light intensity data using the time-series error data, wherein said range is referred to as an abnormality occurrence range,
          calculate a feature-amount regarding the time-series error data in the calculated abnormality occurrence range ,
          compare the calculated feature amount with a predetermined threshold value, and determine whether the type of abnormality is a two-step reaction abnormality or a drift abnormality or another abnormality;

wherein said feature amount regarding the time-series error data is one of the following:

(i) the number of data points included in the abnormality occurrence range;
(ii) a value that can be calculated from the error, such as an average value, a dispersion value, a total value, a maximum value, a median value, a quartile value, an error value of a start position, or an error value of an end position of the time-series error data within the abnormality occurrence range, a time at which the maximum value, the median value, or the quartile value is taken, a value calculated by combining the plurality of values and using four arithmetic operations;
(iii) a parameter obtained by approximating the approximation function, an index indicating a degree of the approximation; or
(iv) a value calculated by combining the feature amounts calculated in each range of a plurality of ranges obtained by dividing the abnormality occurrence range and using four arithmetic operations.

2. The automatic analytical apparatus according to claim 1,
wherein the control unit (121) is configured to
normalize the deviation feature information on the basis of information obtained from at least one of the light intensity data and the approximate curve, and
detect and classify the abnormality using the normalized deviation feature information.

3. The automatic analytical apparatus according to claim 2,
wherein the control unit (121) is configured to
normalize information on time included in the deviation feature information on the basis of information on time obtained from at least one of the light intensity data and the approximate curve, and
normalize information on the light intensity included in the deviation feature information on the basis of information on the light intensity obtained from at least one of the light intensity data and the approximate curve.

4. The automatic analytical apparatus according to claim 1,
wherein the control unit (121) is configured to
take the light intensity data in a predetermined period,
calculate deviation feature information based on deviation information between the light intensity data in the period and the approximate curve, and
detect and classify abnormality included in the light intensity data using the deviation feature information.

5. The automatic analytical apparatus according to claim 4,
wherein the control unit (121) is configured to determine whether the measurement in the measurement unit (115, 116) stops or continues on the basis of the classified type of abnormality.

6. The automatic analytical apparatus according to claim 1,
wherein the approximation function is a function having
a first area in which a change amount of the light intensity with respect to a time gradually increases, and
a second area in which a change amount of the light intensity with respect to the time is smaller than that in the first area at a time after the first area.

7. The automatic analytical apparatus according to claim 1,
wherein the output unit (118, 124) is configured to output at least one of:

(1) the light intensity data output using time as a first axis and a light intensity value as a second axis,
(2) the approximate curve output using time as a first axis and a light intensity value as a second axis,
(3) the deviation feature information,
(4) a result of analysis,
(5) an equation of the approximation function,
(6) a result of detection of an abnormality,
(7) a result of classification of an abnormality, and
(8) information on a process of coping with the abnormality.

8. The automatic analytical apparatus according to claim 1,

wherein a blood coagulation reaction is analyzed.

9. The automatic analytical apparatus according to claim 1,
   wherein the control unit (121) is configured to classify the deviation feature information on the basis of a decision tree stored in the storage unit (119).

10. The automatic analytical apparatus according to claim 1,
    wherein the storage unit (119) is configured to store any one of:

    (1) at least one any-dimensional vector associated with the type of abnormality,
    (2) an identification boundary in at least one any-dimensional space associated with the type of abnormality,
    (3) a partial space in at least one any-dimensional space associated with the type of abnormality, and
    (4) an evaluation function in which an any-dimensional vector is used as an argument and a value associated with the type of abnormality is used as an output value, and

    the control unit (121) is configured to
    convert the deviation feature information into any-dimensional vector feature information through linear or non-linear mapping, and
    classify the type of abnormality on the basis of the any-dimensional vector, the identification boundary, the partial space, or the output value of the evaluation function.

11. The automatic analytical apparatus according to claim 1,
    wherein the abnormality includes at least one of:

    (1) an abnormality in which the amount of change in light intensity value in the light intensity data suddenly increases or decreases,
    (2) an abnormality in which the amount of change in light intensity value in the light intensity data once increases and decreases, and then, increases again, and
    (3) an abnormality in which the amount of change in light intensity value in the light intensity data remains at a certain value or in a predetermined range.

**Patentansprüche**

1. Automatische analytische Vorrichtung, die Folgendes umfasst:

   einen Reaktionsbehälter (104) zum Mischen einer Probe mit einem Reagenz, um eine Reaktionslösung zu bilden;
   eine Messeinheit (115, 116), die konfiguriert ist, eine Reaktionslösung in dem Reaktionsbehälter mit Licht zu bestrahlen und eine Zeitreihe der Stärke von durchgelassenem Licht oder gestreutem Licht zu messen;
   eine Steuereinheit (121), die konfiguriert ist, die Zeitreihenlichtstärkedaten, die durch die Messung in der Messeinheit erhalten werden, zu verarbeiten,
   eine Speichereinheit (119), die konfiguriert ist, eine oder mehrere Näherungsfunktionen, die jeweils eine Zeitreihenänderung der Lichtstärkedaten approximieren, zu speichern; und
   eine Ausgabeeinheit (118, 124), die konfiguriert ist, ein Verarbeitungsergebnis der Steuereinheit (121) auszugeben, wobei die Ausgabeeinheit (118, 124) konfiguriert ist, Informationen über eine detektierte und eingeordnete Unregelmäßigkeit auszugeben;
   wobei
   die Steuereinheit (121) konfiguriert ist zum:

   Auswählen einer der Näherungsfunktionen, die in der Speichereinheit (119) gespeichert sind,
   Berechnen einer Näherungskurve, die eine Zeitreihenänderung der Lichtstärkedaten angibt, unter Verwendung der ausgewählten Näherungsfunktion,
   Berechnen von Abweichungsmerkmalsinformationen anhand von Abweichungsinformationen zwischen den Lichtstärkedaten und der Näherungskurve, wobei die Abweichungsmerkmalsinformationen als Informationen definiert sind, die einen Grad einer Näherung der Näherungskurve an die Zeitreihenlichtstärkedaten repräsentieren; und
   Detektieren und Einordnen einer Unregelmäßigkeit, die in den Lichtstärkedaten enthalten ist, unter Verwendung von Zeitreihenfehlerdaten als die Abweichungsmerkmalsinformationen, wobei die Steuereinheit (121) konfiguriert ist, die Zeitreihenfehlerdaten, die eine Zeitreihenänderung eines Fehlers zwischen den Lichtstärkedaten und der Näherungskurve angeben, zu berechnen,

wobei der Fehler der Unterschied zwischen der Lichtstärke, die unter Verwendung der Näherungskurve berechnet wird, und den Zeitreihenlichtstärkedaten ist;
**dadurch gekennzeichnet, dass**,
um zu bestimmen, ob in den Lichtstärkedaten eine Anomalie enthalten ist, die Steuereinheit (121) konfiguriert ist zum:

Einstellen eines Schwellenfehlerwerts, der ein Schwellenwert eines Fehlers für die Zeitreihenfehlerdaten ist, Berechnen der Anzahl von Datenpunkten mit einem Fehler, der gleich dem Schwellenfehlerwert oder größer als dieser ist, und Vergleichen der berechneten Anzahl von Datenpunkten mit einem Schwellenwert für die Anzahl von Unregelmäßigkeitsdatenpunkten, um zu bestimmen, ob eine Unregelmäßigkeit vorhanden ist; und, um die Unregelmäßigkeit einzuordnen, die Steuereinheit (121) konfiguriert ist zum:

Bestimmen, ob es eine plötzliche Änderung der Zeitreihenfehlerdaten gibt, wobei die plötzliche Änderung durch Einstellen eines Schwellenwerts in dem Änderungsbetrag zwischen einem Fehler eines bestimmten Datenpunkts und einem Fehler eines folgenden Datenpunkts detektiert wird, und dann, wenn eine plötzliche Änderung vorhanden ist, Einordnen der detektierten Unregelmäßigkeit als eine Sprungunregelmäßigkeit und dann, wenn eine plötzliche Änderung nicht vorhanden ist:

Berechnen eines Bereichs in der Zeitreihe, in dem die Unregelmäßigkeit in den Zeitreihenlichtstärkedaten auftritt, unter Verwendung der Zeitreihenfehlerdaten, wobei auf den Bereich als einen Unregelmäßigkeitsereignisbereich Bezug genommen wird,
Berechnen eines Merkmalsbetrags bezüglich der Zeitreihenfehlerdaten in dem berechneten Unregelmäßigkeitsereignisbereich,
Vergleichen des berechneten Merkmalsbetrags mit einem vorbestimmten Schwellenwert und Bestimmen, ob der Typ der Unregelmäßigkeit eine Zweistufenreaktionsunregelmäßigkeit oder eine Driftunregelmäßigkeit oder eine andere Unregelmäßigkeit ist;

wobei der Merkmalsbetrag bezüglich der Zeitreihenfehlerdaten einer der Folgenden ist:

(i) die Anzahl von Datenpunkten, die in dem Unregelmäßigkeitsereignisbereich enthalten sind;
(ii) ein Wert, der aus dem Fehler berechnet werden kann, wie etwa ein Durchschnittswert, ein Dispersionswert, ein Gesamtwert, ein maximaler Wert, ein Mittelwert, ein Quartilswert, ein Fehlerwert einer Startposition oder ein Fehlerwert einer Endposition der Zeitreihenfehlerdaten innerhalb des Unregelmäßigkeitsereignisbereichs, eine Zeit, zu der der maximale Wert, der Mittelwert oder der Quartilswert aufgenommen wird, ein Wert, der durch Vereinigen der mehreren Werte und unter Verwendung vier arithmetischer Operationen berechnet wird;
(iii) ein Parameter, der durch Annähern der Näherungsfunktion erhalten wird, wobei ein Index einen Grad der Näherung angibt; oder
(iv) einen Wert, der durch Vereinigen der Merkmalsbeträge, die in jedem Bereich mehrerer Bereiche, die durch Unterteilen des Unregelmäßigkeitsereignisbereichs erhalten werden, und unter Verwendung von vier arithmetischen Operationen berechnet werden.

2. Automatische analytische Vorrichtung nach Anspruch 1,
wobei die Steuereinheit (1221) konfiguriert ist zum:

Normieren der Abweichungsmerkmalsinformationen anhand von Informationen, die aus den Lichtstärkedaten und/oder der Näherungskurve erhalten werden, und
Detektieren und Einordnen der Unregelmäßigkeit unter Verwendung der normierten Abweichungsmerkmalsinformationen.

3. Automatische analytische Vorrichtung nach Anspruch 2,
wobei die Steuereinheit (121) konfiguriert ist zum:

Normieren von Informationen über eine Zeit, die in den Abweichungsmerkmalsinformationen enthalten sind, anhand von Informationen über eine Zeit, die aus den Lichtstärkedaten und/oder der Näherungskurve erhalten werden, und

Normieren von Informationen über die Lichtstärke, die in den Abweichungsmerkmalsinformationen enthalten sind, anhand von Informationen über die Lichtstärke, die aus den Lichtstärkedaten und/oder der Näherungskurve erhalten werden.

4. Automatische analytische Vorrichtung nach Anspruch 1,
   wobei die Steuereinheit (121) konfiguriert ist zum:

   Aufnehmen der Lichtstärkedaten in einer vorbestimmten Periode,
   Berechnen von Abweichungsmerkmalsinformationen anhand von Abweichungsinformationen zwischen den Lichtstärkedaten in der Periode und der Näherungskurve und
   Detektieren und Einordnen der Unregelmäßigkeit, die in den Lichtstärkedaten enthalten ist, unter Verwendung der Abweichungsmerkmalsinformationen.

5. Automatische analytische Vorrichtung nach Anspruch 4,
   wobei die Steuereinheit (121) konfiguriert ist, anhand des eingeordneten Typs der Unregelmäßigkeit zu bestimmen, ob die Messung in der Messeinheit (115, 116) aufhört oder weitergeht.

6. Automatische analytische Vorrichtung nach Anspruch 1,
   wobei die Näherungsfunktion eine Funktion ist mit:

   einem ersten Bereich, in dem ein Änderungsbetrag der Lichtstärke in Bezug auf eine Zeit allmählich zunimmt, und einem zweiten Bereich, in dem ein Änderungsbetrag der Lichtstärke in Bezug auf die Zeit kleiner als der in dem ersten Bereich und zu einer Zeit nach dem ersten Bereich ist.

7. Automatische analytische Vorrichtung nach Anspruch 1,
   wobei die Ausgabeeinheit (118, 124) konfiguriert ist, mindestens eines auszugeben von:

   (1) den Lichtstärkedaten, die unter Verwendung der Zeit als einer ersten Achse und einem Lichtstärkewert als einer zweiten Achse ausgegeben werden,
   (2) der Näherungskurve, die unter Verwendung der Zeit als einer ersten Achse und einem Lichtstärkewert als einer zweiten Achse ausgegeben werden,
   (3) den Abweichungsmerkmalsinformationen,
   (4) einem Analyseergebnis,
   (5) einer Gleichung der Näherungsfunktion,
   (6) einem Ergebnis einer Detektion einer Unregelmäßigkeit,
   (7) einem Ergebnis einer Einordnung einer Unregelmäßigkeit und
   (8) Informationen über einen Prozess des Zurechtkommens mit der Unregelmäßigkeit.

8. Automatische analytische Vorrichtung nach Anspruch 1,
   wobei eine Blutkoagulierungsreaktion analysiert wird.

9. Automatische analytische Vorrichtung nach Anspruch 1,
   wobei die Steuereinheit (121) konfiguriert ist, die Abweichungsmerkmalsinformationen anhand eines Entscheidungsbaums, der in der Speichereinheit (119) gespeichert ist, einzuordnen.

10. Automatische analytische Vorrichtung nach Anspruch 1,
    wobei die Speichereinheit (119) konfiguriert ist, eines der Folgenden zu speichern:

    (1) mindestens einen Vektor beliebiger Dimension, der dem Typ der Unregelmäßigkeit zugeordnet ist,
    (2) eine Identifikationsgrenze in mindestens einem Raum beliebiger Dimension, der dem Typ der Unregelmäßigkeit zugeordnet ist, und
    (3) einen Teilraum in mindestens einem Raum beliebiger Dimension, der dem Typ der Unregelmäßigkeit zugeordnet ist, und
    (4) eine Beurteilungsfunktion, in der ein Vektor beliebiger Dimension als ein Argument verwendet wird und ein Wert, der dem Typ der Unregelmäßigkeit zugeordnet ist, als ein Ausgabewert verwendet wird, und
    die Steuereinheit (121) konfiguriert ist zum:

    Umsetzen der Abweichungsmerkmalsinformationen in Merkmalsinformationen eines Vektors beliebiger Dimension durch eine lineare oder nicht lineare Abbildung und

Einordnen des Typs der Unregelmäßigkeit anhand des Vektors beliebiger Dimension, der Identifikationsgrenze, des Teilraums oder des Ausgabewerts der Beurteilungsfunktion.

**11.** Automatische analytische Vorrichtung nach Anspruch 1,
wobei die Unregelmäßigkeit mindestens eine der folgenden Unregelmäßigkeiten enthält:

(1) eine Unregelmäßigkeit, in der der Änderungsbetrag des Lichtstärkewerts in den Lichtstärkedaten plötzlich zunimmt oder abnimmt,

(2) eine Unregelmäßigkeit, in der der Änderungsbetrag des Lichtstärkewerts in den Lichtstärkedaten einmal zunimmt und abnimmt und dann wieder zunimmt, und

(3) eine Unregelmäßigkeit, in der der Änderungsbetrag des Lichtstärkewerts in den Lichtstärkedaten auf einem bestimmten Wert oder in einem bestimmten Bereich bleibt.

**Revendications**

1. Appareil d'analyse automatique, comprenant :

un récipient de réaction (104) pour mélanger un échantillon avec un réactif afin de former une solution de réaction ;
une unité de mesure (115, 116) qui est configurée pour irradier une solution de réaction dans le récipient de réaction avec de la lumière et pour mesurer une série temporelle de l'intensité de la lumière transmise ou de la lumière diffusée ;
une unité de commande (121) qui est configurée pour traiter les données d'intensité lumineuse de série temporelle obtenues par la mesure dans l'unité de mesure ;
une unité de stockage (119) qui est configurée pour stocker une ou plusieurs fonctions d'approximation approximant chacune un changement de série temporelle dans les données d'intensité lumineuse ; et
une unité de sortie (118, 124) qui est configurée pour délivrer un résultat de traitement de l'unité de commande (121), l'unité de sortie (118, 124) étant configurée pour délivrer des informations sur une anomalie détectée et classifiée ;
dans lequel
l'unité de commande (121) est configurée pour

sélectionner l'une quelconque des fonctions d'approximation stockées dans l'unité de stockage (119),
calculer une courbe approximative indiquant un changement de série temporelle dans les données d'intensité lumineuse en utilisant la fonction d'approximation sélectionnée,
calculer des informations de caractéristique d'écart sur la base d'informations d'écart entre les données d'intensité lumineuse et la courbe approximative, les informations de caractéristique d'écart étant définies comme des informations représentant un degré d'approximation de la courbe approximative par rapport aux données d'intensité lumineuse de série temporelle ; et
détecter et classifier une anomalie incluse dans les données d'intensité lumineuse en utilisant des données d'erreur de série temporelle comme informations de caractéristique d'écart, l'unité de commande (121) étant configurée pour calculer les données d'erreur de série temporelle indiquant un changement de série temporelle dans une erreur entre les données d'intensité lumineuse et la courbe approximative, ladite erreur étant la différence entre l'intensité lumineuse calculée en utilisant la courbe approximative et les données d'intensité lumineuse de série temporelle ;

**caractérisé en ce que**,
pour déterminer si une anomalie est incluse dans les données d'intensité lumineuse, l'unité de commande (121) est configurée pour :
définir une valeur d'erreur seuil, qui est une valeur seuil d'une erreur pour les données d'erreur de série temporelle, calculer le nombre de points de données ayant une erreur égale ou supérieure à la valeur d'erreur seuil, et comparer le nombre calculé de points de données avec une valeur seuil pour le nombre de points de données d'anomalie pour déterminer si une anomalie est présente ; et pour classifier l'anomalie, l'unité de commande (121) est configurée pour :
déterminer s'il y a un changement soudain dans les données d'erreur de série temporelle, ledit changement soudain étant détecté en définissant une valeur seuil dans la quantité de changement entre une erreur d'un certain point de données et une erreur d'un point de données subséquent et, si un changement soudain est présent, classifier l'anomalie détectée comme une anomalie de saut et, si un changement soudain n'est pas présent :

calculer une plage dans la série temporelle dans laquelle l'anomalie se produit dans les données d'intensité lumineuse de série temporelle en utilisant les données d'erreur de série temporelle, ladite plage étant appelée plage d'occurrence d'anomalie,

calculer une quantité caractéristique concernant les données d'erreur de série temporelle dans la plage d'occurrence d'anomalie calculée,

comparer la quantité caractéristique calculée avec une valeur seuil prédéterminée et déterminer si le type d'anomalie est une anomalie de réaction en deux étapes, une anomalie de dérive ou une autre anomalie ; ladite quantité caractéristique concernant les données d'erreur de série temporelle étant l'une des suivantes :

(i) le nombre de points de données inclus dans la plage d'occurrence d'anomalie ;

(ii) une valeur qui peut être calculée à partir de l'erreur, telle qu'une valeur moyenne, une valeur de dispersion, une valeur totale, une valeur maximale, une valeur médiane, une valeur de quartile, une valeur d'erreur d'une position de départ ou une valeur d'erreur d'une position de fin des données d'erreur de série temporelle dans la plage d'occurrence d'anomalie, un instant auquel la valeur maximale, la valeur médiane ou la valeur de quartile est prise, une valeur calculée en combinant la pluralité de valeurs et en utilisant quatre opérations arithmétiques ;

(iii) un paramètre obtenu en approximant la fonction d'approximation, un indice indiquant un degré de l'approximation ; ou

(iv) une valeur calculée en combinant les quantités caractéristiques calculées dans chaque plage d'une pluralité de plages obtenues en divisant la plage d'occurrence d'anomalie et en utilisant quatre opérations arithmétiques.

2. Appareil d'analyse automatique selon la revendication 1,
dans lequel l'unité de commande (121) est configurée pour
normaliser les informations de caractéristique d'écart sur la base des informations obtenues à partir des données d'intensité lumineuse et/ou de la courbe approximative, et
détecter et classifier l'anomalie en utilisant les informations de caractéristique d'écart normalisées.

3. Appareil d'analyse automatique selon la revendication 2,
dans lequel l'unité de commande (121) est configurée pour
normaliser les informations sur le temps incluses dans les informations de caractéristique d'écart sur la base d'informations sur le temps obtenues à partir des données d'intensité lumineuse et/ou de la courbe approximative, et
normaliser les informations sur l'intensité lumineuse incluses dans les informations de caractéristique d'écart sur la base d'informations sur l'intensité lumineuse obtenues à partir des données d'intensité lumineuse et/ou de la courbe approximative.

4. Appareil d'analyse automatique selon la revendication 1,
dans lequel l'unité de commande (121) est configurée pour prendre les données d'intensité lumineuse dans une période prédéterminée, calculer des informations de caractéristique d'écart sur la base d'informations d'écart entre les données d'intensité lumineuse dans la période et la courbe approximative, et
détecter et classifier une anomalie incluse dans les données d'intensité lumineuse en utilisant les informations de caractéristique d'écart.

5. Appareil d'analyse automatique selon la revendication 4,
dans lequel l'unité de commande (121) est configurée pour déterminer si la mesure dans l'unité de mesure (115, 116) s'arrête ou continue sur la base du type d'anomalie classifié.

6. Appareil d'analyse automatique selon la revendication 1,
dans lequel la fonction d'approximation est une fonction ayant
une première zone dans laquelle une quantité de changement de l'intensité lumineuse par rapport à un temps augmente progressivement, et
une seconde zone dans laquelle une quantité de changement de l'intensité lumineuse par rapport au temps est plus petite que celle dans la première zone à un temps postérieur à la première zone.

**7.** Appareil d'analyse automatique selon la revendication 1,
dans lequel l'unité de sortie (118, 124) est configurée pour délivrer au moins l'une des sorties suivantes :

(1) la sortie de données d'intensité lumineuse en utilisant le temps comme premier axe et une valeur d'intensité lumineuse comme second axe,
(2) la sortie de courbe approximative en utilisant le temps comme premier axe et une valeur d'intensité lumineuse comme second axe,
(3) les informations de caractéristique d'écart,
(4) un résultat d'analyse,
(5) une équation de la fonction d'approximation,
(6) un résultat de détection d'une anomalie,
(7) un résultat de classification d'une anomalie et
(8) des informations sur un processus de traitement de l'anomalie.

**8.** Appareil d'analyse automatique selon la revendication 1,
dans lequel une réaction de coagulation du sang est analysée.

**9.** Appareil d'analyse automatique selon la revendication 1,
dans lequel l'unité de commande (121) est configurée pour classifier les informations de caractéristique d'écart sur la base d'un arbre de décision stocké dans l'unité de stockage (119).

**10.** Appareil d'analyse automatique selon la revendication 1,
dans lequel l'unité de stockage (119) est configurée pour stocker l'un quelconque des éléments suivants :

(1) au moins un vecteur de dimension quelconque associé au type d'anomalie,
(2) une limite d'identification dans au moins un espace de dimension quelconque associé au type d'anomalie,
(3) un espace partiel dans au moins un espace de dimension quelconque associé au type d'anomalie et
(4) une fonction d'évaluation dans laquelle un vecteur de dimension quelconque est utilisé comme argument et une valeur associée au type d'anomalie est utilisée comme valeur de sortie, et

l'unité de commande (121) est configurée pour
convertir les informations de caractéristique d'écart en informations de caractéristique de vecteur de dimension quelconque par mappage linéaire ou non linéaire et
classifier le type d'anomalie sur la base du vecteur de dimension quelconque, de la limite d'identification, de l'espace partiel ou de la valeur de sortie de la fonction d'évaluation.

**11.** Appareil d'analyse automatique selon la revendication 1,
dans lequel l'anomalie inclut au moins l'une des suivantes :

(1) une anomalie dans laquelle la quantité de changement de la valeur d'intensité lumineuse dans les données d'intensité lumineuse augmente ou diminue soudainement,
(2) une anomalie dans laquelle la quantité de changement de la valeur d'intensité lumineuse dans les données d'intensité lumineuse augmente et diminue une fois, puis augmente à nouveau, et
(3) une anomalie dans laquelle la quantité de changement de la valeur d'intensité lumineuse dans les données d'intensité lumineuse reste à une certaine valeur ou dans une plage prédéterminée.

# Fig. 1

# Fig. 2

```
                        ┌──────────────────────────┐
                        │          Start           │
                        └──────────────────────────┘
                                     │
            ┌─────────────────────────────────────────┐
            │       Acquire approximation function     │────  S201
            └─────────────────────────────────────────┘
                                     │
            ┌─────────────────────────────────────────┐
            │      Take time-series light intensity data │────  S202
            └─────────────────────────────────────────┘
                                     │
            ┌─────────────────────────────────────────┐
            │        Calculate approximate curve       │────  S203
            └─────────────────────────────────────────┘
                                     │
            ┌─────────────────────────────────────────┐
            │    Calculate deviation feature information │────  S204
            └─────────────────────────────────────────┘
                                     │
            ┌─────────────────────────────────────────┐
            │   Calculate reaction curve feature amount │────  S205
            └─────────────────────────────────────────┘
                                     │
            ┌─────────────────────────────────────────┐
            │      Determine abnormality on basis of   │────  S206
            │        deviation feature information      │
            └─────────────────────────────────────────┘
                                     │
  NO                               ◇────────────────────── S207
  ┌────────────────────────< Does abnormality occur? >
  │                                 ◇
  │                                 │ YES
  │         ┌─────────────────────────────────────────┐
  │         │   Classify abnormality based on deviation │────  S208
  │         │            feature information           │
  │         └─────────────────────────────────────────┘
  │                                 │
  │         ┌─────────────────────────────────────────┐
  │         │          Notify of abnormality          │────  S209
  │         └─────────────────────────────────────────┘
  │                                 │
  └─────────────────────────────────┤
                                     │
                        ┌──────────────────────────┐
                        │           End            │
                        └──────────────────────────┘
```

# Fig. 3

```
                          ┌──────────────┐
                          │    Start     │
                          └──────┬───────┘
                                 │
                                 ▼                    S301
              YES        ◇ Is there sudden change ◇
       ┌─────────────────   in error data?
       │                          │
       │                         NO
       │                          ▼
       │              ┌─────────────────────────┐
       │              │  Calculate abnormality   │── S302
       │              │   occurrence range       │
       │              └────────────┬─────────────┘
       │              ┌─────────────────────────────┐
       │              │  Calculate abnormality       │── S303
       │              │ occurrence range feature amount│
       │              └────────────┬────────────────┘
       │                           ▼              S304
       │               ◇ Is abnormality              ◇
       │          occurrence range frequent in second    YES
       │                 half of reaction?
```

Is there sudden change in error data? — S301

Calculate abnormality occurrence range — S302

Calculate abnormality occurrence range feature amount — S303

Is abnormality occurrence range frequent in second half of reaction? — S304

Is abnormality occurrence range frequent in first half of reaction? — S305

Is feature amount equal to or greater than threshold value? — S306

Is feature amount equal to or greater than threshold value? — S307

Classify jump abnormality — S308

Classify two-step reaction abnormality — S309

Classify other abnormality — S310

Classify drift abnormality — S311

End

# Fig. 4

```
                    ┌─────────────────────────┐
                    │          Start          │
                    └─────────────────────────┘
                                 │
            ┌────────────────────────────────────┐
            │    Acquire approximation function   │───∽ S201
            └────────────────────────────────────┘
                                 │
            ┌────────────────────────────────────┐
            │   Take time-series light intensity  │───∽ S202
            │                data                 │
            └────────────────────────────────────┘
                                 │
            ┌────────────────────────────────────┐
            │      Calculate approximate curve    │───∽ S203
            └────────────────────────────────────┘
                                 │
            ┌────────────────────────────────────┐
            │  Calculate deviation feature info   │───∽ S204
            └────────────────────────────────────┘
                                 │
            ┌────────────────────────────────────┐
            │ Calculate reaction curve feature    │───∽ S205
            │              amount                 │
            └────────────────────────────────────┘
                                 │
            ┌────────────────────────────────────┐
            │ Normalize deviation feature info    │───∽ S401
            └────────────────────────────────────┘
                                 │
            ┌────────────────────────────────────┐
            │   Determine abnormality on basis of │───∽ S206
            │      deviation feature information  │
            └────────────────────────────────────┘
                                 │
                                 ▼
      NO              ◇ Does abnormality occur? ◇───∽ S207
       │                         │
       │                        YES
       │            ┌────────────────────────────────────┐
       │            │ Classify abnormality based on       │───∽ S208
       │            │    deviation feature information     │
       │            └────────────────────────────────────┘
       │                         │
       │            ┌────────────────────────────────────┐
       │            │        Notify of abnormality        │───∽ S209
       │            └────────────────────────────────────┘
       │                         │
       └────────────────────────►│
                                 ▼
                    ┌─────────────────────────┐
                    │           End           │
                    └─────────────────────────┘
```

# Fig. 5

```
                    ┌─────────────────────┐
                    │        Start        │
                    └──────────┬──────────┘
                               │
              ┌────────────────────────────────┐
              │  Acquire approximation function │──── S201
              └────────────────┬───────────────┘
                               │
              ┌────────────────────────────────┐
              │  Take time-series light intensity │──── S501
              │              data              │
              └────────────────┬───────────────┘
                               │
              ┌────────────────────────────────┐
              │   Calculate approximate curve   │──── S203
              └────────────────┬───────────────┘
                               │
              ┌────────────────────────────────┐
              │   Calculate deviation feature   │──── S204
              │           information           │
              └────────────────┬───────────────┘
                               │
              ┌────────────────────────────────┐
              │ Calculate reaction curve feature│──── S205
              │             amount              │
              └────────────────┬───────────────┘
                               │
              ┌────────────────────────────────┐
              │   Normalize deviation feature   │──── S401
              │           information           │
              └────────────────┬───────────────┘
                               │
              ┌────────────────────────────────┐
              │ Determine abnormality on basis  │──── S206
              │ of deviation feature information │
              └────────────────┬───────────────┘
```

S207

Does abnormality occur?   YES

NO

Classify abnormality based on deviation feature information ──── S208

Reaction end determination ──── S502

Notify of abnormality ──── S504

S503

Has reaction ended?

NO

S505

Abnormality for which measurement is to be ended?

NO

YES

End

YES

End

Fig. 6

Fig. 7

# Fig. 8

Light intensity

602

801

802

803

601

Time

# Fig. 9

Error

701

901

902

903

d

e

601

Time

803

# Fig. 10

# Fig. 11

## Fig. 12

## Fig. 13

# Fig. 14

Analysis result                                                              1401

| Sample number | Test item | Test value | Abnormality code |
|---------------|-----------|------------|------------------|
| 0001 | Fbg | XXX | 000 |
| 0002 | Fbg | YYY | 000 |
| 0003 | APTT | ZZZ | 001 |
| ... | | | |

1402

Sample number:0003

— Approximate curve

■ ■ ■ ■ Light intensity data

# Fig. 15

# Fig. 16

## Fig. 17

## Fig. 18

# Fig. 19

# Fig. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003169700 A
- EP 2711713 A1
- EP 2434292 A1
- EP 2594944 A1